# EUROPEAN PATENT APPLICATION

(11) **EP 3 381 931 A1**
(43) Date of publication of application: **03.10.2018**
(21) Application number: 17163566.7
(22) Date of filing: 29.03.2017
(51) Int. Cl.: C07K 14/235, A61K 39/02

(54) **ADENYLATE CYCLASE TOXOID WITH REDUCED CYTOLYTIC ACTIVITY**

(71) Applicant: Mikrobiologicky ustav AV CR, v.v.i., 142 20 Praha 4 - Krc (CZ)
(72) Inventor: Masin, Jiri, 25082 Uvaly (CZ); Osicka, Radim, 14000 Praha 4 (CZ); Sebo, Peter, 14000 Praha 4 (CZ); Osickova, Adriana, 14000 Praha 4 (CZ)
(74) Representative: Hartvichova, Katerina

(57) **Abstract**

The present invention provides a polypeptide antigen comprising amino acid sequence of adenylate cyclase protein of *Bordetella pertussis* bearing the following mutations:
(i) substitution of the alanine residue in position 680 by a proline residue, and
(ii) substitution of the valine residue in position 695 by a proline residue,
or one or more fragments thereof, wherein said fragments comprise at least amino acids from position 374 to position 400, from position 500 to position 700, and from position 860 to position 1706.

Said polypeptide antigen is suitable for use in a method of treatment and/or prevention of a disease caused by *Bordetella pertussis,* in particular against pertussis or whooping cough disease.

The invention further provides a chimeric protein, a polynucleotide encoding the polypeptide antigen or chimeric protein, a vector and a host cell for the production of said polypeptide or protein.

## Description

### Field of Art

The present invention relates to a polypeptide antigen derived from the adenylate cyclase toxin (ACT, AC-Hly or CyaA) of *Bordetella pertussis* bearing mutations decreasing its cytolytic activity.

### Background Art

*Bordetella pertussis,* the causative agent of whooping cough, secretes an adenylate cyclase toxin-hemolysin (CyaA, ACT or AC-Hly), a member of the repeat in toxin (RTX) protein family that plays a crucial role in the early stages of bacterial colonization of human respiratory tract. CyaA targets and annihilates bactericidal activities of phagocytes, such as oxidative burst and complement- or antibody-mediated opsonophagocytic uptake and killing of bacteria. Through cAMP signaling, CyaA skews also TLR-triggered maturation of dendritic cells, inhibiting pro-inflammatory IL-12 and TNF-α secretion and enhancing IL-10 production and Treg expansion, likely hampering also induction of adaptive immune responses to *Bordetella* infections.

Action of the adenylate cyclase toxin, playing a critical role in the early phases of bacterial colonization, appears to be of crucial importance for suppression of host innate immune mechanisms, as well as for modulation of adaptive T and B cell responses and for evasion of host immunity. The absence of CyaA in the current acellular pertussis (aP) vaccine formulation and the inability of such vaccines to induce CyaA-neutralizing antibodies represents one of the key factors contributing to the failure of the aP vaccines in conferring a long-lasting immune protection against whooping cough in highly vaccinated populations.

There is an urgent public health need to improve the composition of the aP vaccine so as to enhance the protection of aP-vaccinated subjects against colonization by the pathogen and block its transmission. Given the protective immunogenicity of CyaA and its major immunosuppressive role played in *Bordetella* infections, CyaA toxoids are a prime candidate for inclusion into the next generation of improved aP vaccines.

CyaA is a 1,706-residue-long (177-kDa) bifunctional protein that consists of an amino-terminal adenylate cyclase (AC) domain of about 400 residues and of an RTX hemolysin moiety (Hly) of about 1,300 residues. The Hly moiety inserts into cellular membranes and mediates translocation of the enzymatic AC domain into cell cytosol, where this binds calmodulin and catalyzes conversion of ATP to cAMP, thus subverting cellular signaling.

In parallel, the Hly can form small cation-selective membrane pores that permeabilize target cell membrane and allow efflux of potassium ions from cells, thus causing colloid-osmotic cell lysis, such as hemolysis of erythrocytes.

The capacity of CyaA to tightly associate with target cell membrane, translocate the AC domain into cells and form membrane pores depends on a posttranslational activation step that occurs during synthesis of CyaA inside producing bacteria. The activation consists in covalent attachment of fatty acyl residues, typically of palmitic residues (C16:0), by an amide bond onto the ε-amino groups of the side chains of internal lysine residues 860 (K860) and 983 (K983) of CyaA. Acylation of K983 is then essential for biological activity of CyaA as toxin penetrating mammalian cells. The acylation reaction is catalyzed by the protein toxin acyltransferase CyaC, which is produced in the bacteria together with CyaA and uses the acyl-acyl carrier protein (acyl-ACP) as donor of the acyl residues.

Recently, the construction of a doubly detoxified CyaA antigen, which besides lacking the AC enzyme activity is also devoid of most of its pore-forming activity accounting for the hemolytic properties of CyaA, was achieved by the combination of substitution of the glutamate 570 residue by a charge-neutralizing glutamine residue (E570Q) in the pore-forming domain with the substitution of the lysine residue 860 in the acylated domain by a charge-conserving arginine residue substitution (K860R), respectively (EP2233569, EP2411513). On the contrary, toxins and toxoids exhibiting a specifically enhanced pore-forming capacity, due to combination of substitutions of negatively charged residues in the AC-to-Hly linking segments by neutral hydrophilic residues, such as the CyaA-D445N+D446N+E448Q-AC⁻, are described in EP16001877.

### Disclosure of the Invention

The present invention relates to a novel type of CyaA constructs having the specific pore-forming activity selectively reduced due to substitutions of the alanine 680 and of valine 695 residues in the pore-forming domain by α-helix-breaking proline residues (A680P+V695P) and therefore exhibiting a selectively reduced capacity to permeabilize artificial and natural membranes and lyze cells. These constructs are further characterized by an intact capacity to form the receptor-binding structure that is required for binding to the CR3 receptor-expressing cells (CR3 - complement receptor 3, the heterodimer CD11b/CD18, α_{M}β₂ integrin or Mac-1), such as macrophage cells and which is required for induction of CyaA-neutralizing antibodies.

The present invention thus provides, in a first aspect, a polypeptide antigen comprising amino acid sequence of adenylate cyclase protein (CyaA) of *Bordetella pertussis,* said amino acid sequence of adenylate cyclase protein bearing the following mutations:
(i) substitution of the alanine residue in position 680 by a proline residue, and
(ii) substitution of the valine residue in position 695 by a proline residue,
or one or more fragments thereof, wherein said fragments comprise at least amino acids from position 374 to position 400, from position 500 to position 700, and from position 860 to position 1706. More preferably, the fragments comprise at least amino acids from position 374 to position 700, and from position 850 (or 860) to position 1706.

CyaA has been described as an amino acid sequence, encoded by the *cyaA* gene (BP0760 of GenBank: BX470248.1, Gene ID: 2664492, Sequence: NC_002929.2 (776228..781348)). Accordingly, when amino acid residues or sequences or fragments thereof, or nucleotides or nucleotide sequences of the CyaA protein of *B. pertussis* are cited in the present invention, their positions are given with respect to the sequence of wild-type CyaA protein, where "CyaA protein" or "wild-type CyaA protein" refers to the amino acid residue sequence of adenylate cyclase toxin protein from *Bordetella pertussis* deposited at UniProtKB/Swiss-Prot database under Acc. No.: P0DKX7.1 (https:Hwww.ncbi.nlm.nih.gov/protein/P0DKX7.1) and shown herein as SEQ ID NO. 1.
SEQ ID NO. 1 - wild-type CyaA protein of *B. pertussis*

1 MQQSHQAGYA NAADRESGIP AAVLDGIKAV AKEKNATLMF RLVNPHSTSL IAEGVATKGL
61 GVHAKSSDWG LQAGYIPVNP NLSKLFGRAP EVIARADNDV NSSLAHGHTA VDLTLSKERL
121 DYLRQAGLVT GMADGVVASN HAGYEQFEFR VKETSDGRYA VQYRRKGGDD FEAVKVIGNA
181 AGIPLTADID MFAIMPHLSN FRDSARSSVT SGDSVTDYLA RTRRAASEAT GGLDRERIDL
241 LWKIARAGAR SAVGTEARRQ FRYDGDMNIG VITDFELEVR NALNRRAHAV GAQDVVQHGT
301 EQNNPFPEAD EKIFVVSATG ESQMLTRGQL KEYIGQQRGE GYVFYENRAY GVAGKSLFDD
361 GLGAAPGVPS GRSKFSPDVL ETVPASPGLR RPSLGAVERQ DSGYDSLDGV GSRSFSLGEV
421 SDMAAVEAAE LEMTRQVLHA GARQDDAEPG VSGASAHWGQ RALQGAQAVA AAQRLVHAIA
481 LMTQFGRAGS TNTPQEAASL SAAVFGLGEA SSAVAETVSG FFRGSSRWAG GFGVAGGAMA
541 LGGGIAAAVG AGMSLTDDAP AGQKAAAGAE IALQLTGGTV ELASSIALAL AAARGVTSGL
601 QVAGASAGAA AGALAAALSP MEIYGLVQQS HYADQLDKLA QESSAYGYEG DALLAQLYRD
661 KTAAEGAVAG VSAVLSTVGA AVSIAAAASV VGAPVAVVTS LLTGALNGIL RGVQQPIIEK
721 LANDYARKID ELGGPQAYFE KNLQARHEQL ANSDGLRKML ADLQAGWNAS SVIGVQTTEI

781 SKSALELAAI TGNADNLKSV DVFVDRFVQG ERVAGQPVVL DVAAGGIDIA SRKGERPALT
841 FITPLAAPGE EQRRRTKTGK SEFTTFVEIV GKQDRWRIRD GAADTTIDLA KVVSQLVDAN
901 GVLKHSIKLD VIGGDGDDVV LANASRIHYD GGAGTNTVSY AALGRQDSIT VSADGERFNV
961 RKQLNNANVY REGVATQTTA YGKRTENVQY RHVELARVGQ LVEVDTLEHV QHIIGGAGND
1021 SITGNAHDNF LAGGSGDDRL DGGAGNDTLV GGEGQNTVIG GAGDDVFLQD LGVWSNQLDG
1081 GAGVDTVKYN VHQPSEERLE RMGDTGIHAD LQKGTVEKWP ALNLFSVDHV KNIENLHGSR
1141 LNDRIAGDDQ DNELWGHDGN DTIRGRGGDD ILRGGLGLDT LYGEDGNDIF LQDDETVSDD
1201 IDGGAGLDTV DYSAMIHPGR IVAPHEYGFG IEADLSREWV RKASALGVDY YDNVRNVENV
1261 IGTSMKDVLI GDAQANTLMG QGGDDTVRGG DGDDLLFGGD GNDMLYGDAG NDTLYGGLGD
1321 DTLEGGAGND WFGQTQAREH DVLRGGDGVD TVDYSQTGAH AGIAAGRIGL GILADLGAGR
1381 VDKLGEAGSS AYDTVSGIEN VVGTELADRI TGDAQANVLR GAGGADVLAG GEGDDVLLGG
1441 DGDDQLSGDA GRDRLYGEAG DDWFFQDAAN AGNLLDGGDG RDTVDFSGPG RGLDAGAKGV
1501 FLSLGKGFAS LMDEPETSNV LRNIENAVGS ARDDVLIGDA GANVLNGLAG NDVLSGGAGD
1561 DVLLGDEGSD LLSGDAGNDD LFGGQGDDTY LFGVGYGHDT IYESGGGHDT IRINAGADQL
1621 WFARQGNDLE IRILGTDDAL TVHDWYRDAD HRVEIIHAAN QAVDQAGIEK LVEAMAQYPD
1681 PGAAAAAPPA ARVPDTLMQS LAVNWR

The minimum requirements for the fragments of CyaA are based on findings published in literature (see, e.g., Sebo P. et al., Expert Rev. Vaccines 13(10), 1215-1227 (2014)). It was shown that deletion constructs of CyaA having the structure of the last approximately 900 residues of CyaA intact and bearing the palmitoylated lysine 983 residue were recognized in immunoblots by sera of *B. pertussis*-infected mice and humans (Betsou et al. 1995, Infect. Immun. 63, pp. 3309-3315). It seems that under *in vivo* conditions, a small number of conformational epitopes in the structure of the receptor-binding domain of CyaA are recognized and induce toxin-neutralizing antibodies. The site on the toxin molecule that binds to the receptor was localized between residues 1166 and 1287 (El Azami El Idrisi et al. 2003 JBC 278, pp. 38514-38521). Phage display screening of an antibody library yielded toxin-neutralizing M1H5 and M2B10 monoclonal antibodies that block toxin binding to its receptor upon binding to epitopes formed within repeat blocks II and III of the toxin molecule between residues 1070 to 1340, thus defining one set of key conformational protective epitopes on the toxin molecule (Wang. et al. 2017, Biochemistry 56, pp. 1324-1336). A construct lacking only the last 217 out of 1706 residues of CyaA did not confer any protective immunity, nor induced any toxin-neutralizing antibodies (Betsou et al. 1995, Infect. Immun. 63, pp. 3309-3315). This highlighted the importance of the C-terminal region for AC toxin structure and activity. Indeed, a protein lacking residues 385-1489, but bearing the last 217 residues could still induce antibodies neutralizing toxin activity in vitro, but failed to confer protective immunity *in vivo*. This was most likely due to the absence of one of the key protective epitopes of CyaA that is located at the junction of the AC domain to the hemolysin moiety, between residues 373-400, to which a monoclonal antibody (3D1) binds and subsequently blocks AC domain translocation into target cells.

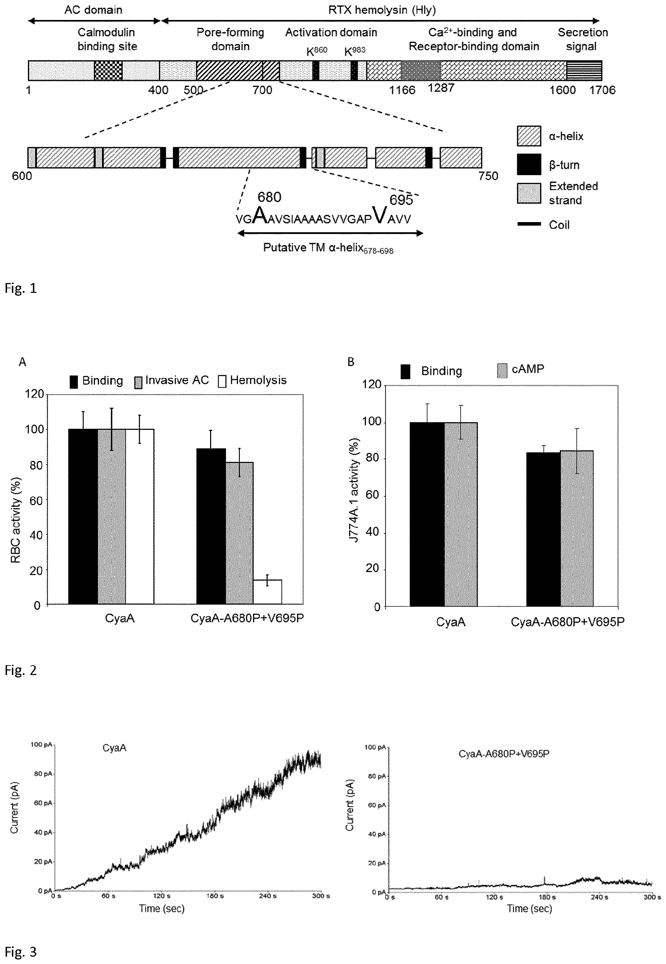

The polypeptide antigen of the present invention comprises a mutated transmembrane segment of the pore-forming domain, comprised within residues 678 and 698. In the framework of the present invention, we have demonstrated that the combination of the A680P and V695P substitutions within the transmembrane α-helix 678 to 698 selectively blocks the pore-forming activity of CyaA, probably by an alteration of pore structure (Fig. 3), while maintaining the ability to induce of CyaA-neutralizing antibodies.
As used herein, the expression "pore-forming domain" refers to the about 200 amino acid sequence following the Hly linking segment in the CyaA protein, preferably it refers to the amino acid residues 500 to 700.

The polypeptide antigen of the present invention may have the N-amino-terminal adenylate cyclase (AC) domain deleted (abbreviated as "AC⁻"). Preferably, the sequence derived from the CyaA protein comprises a sequence derived from the Hly domain of CyaA excluding the part of the AC domain and corresponding, for example, to the amino acids 372 to 1706, 373 to 1706, or 374 to 1706.

Therefore, the present invention preferably provides a polypeptide antigen containing amino acid sequence of adenylate cyclase protein of *Bordetella pertussis* bearing the following mutations:
(i) substitution of the alanine residue in position 680 by a proline residue, and
(ii) substitution of the valine residue in position 695 by a proline residue,
   and having additionally:
(iii) deletion of any or all amino acids in positions 1 to 100-300, or in positions 1 to 100-200, or in positions 1 to 371, or in positions 1 to 372, or in positions 1 to 373, and/or
(iv) insertion of GlySer dipeptide between amino acid residues in positions 188-189 of the amino acid sequence of adenylate cyclase protein.

Preferably, the polypeptide antigen of the invention comprises a sequence bearing the A680P and V695P mutations selected from sequence of amino acid residues from positions 374-1706 of the CyaA protein (SEQ ID NO. 2), sequence of amino acid residues from positions 373-1706 of the CyaA protein (SEQ ID NO. 3), sequence of amino acid residues from positions 372-1706 of the CyaA protein (SEQ ID NO. 4), sequence of amino acid residues from positions 1-1706 of the CyaA protein (SEQ ID NO. 5), sequence of amino acid residues from positions 1-1706 of the CyaA protein with insertion of GlySer dipeptide between the amino acids in positions 188-189 (SEQ ID NO. 6), is provided.
SEQ ID NO. 2 - mutated CyaA polypeptide sequence of fragment 374-1706 bearing A680P and V695P mutations: 
374 KFSPDVL ETVPASPGLR RPSLGAVERQ DSGYDSLDGV GSRSFSLGEV
421 SDMAAVEAAE LEMTRQVLHA GARQDDAEPG VSGASAHWGQ RALQGAQAVA AAQRLVHAIA
481 LMTQFGRAGS TNTPQEAASL SAAVFGLGEA SSAVAETVSG FFRGSSRWAG GFGVAGGAMA
541 LGGGIAAAVG AGMSLTDDAP AGQKAAAGAE IALQLTGGTV ELASSIALAL AAARGVTSGL
601 QVAGASAGAA AGALAAALSP MEIYGLVQQS HYADQLDKLA QESSAYGYEG DALLAQLYRD
661 KTAAEGAVAG VSAVLSTVGP AVSIAAAASV VGAPPAVVTS LLTGALNGIL RGVQQPIIEK
721 LANDYARKID ELGGPQAYFE KNLQARHEQL ANSDGLRKML ADLQAGWNAS SVIGVQTTEI
781 SKSALELAAI TGNADNLKSV DVFVDRFVQG ERVAGQPVVL DVAAGGIDIA SRKGERPALT
841 FITPLAAPGE EQRRRTKTGK SEFTTFVEIV GKQDRWRIRD GAADTTIDLA KVVSQLVDAN
901 GVLKHSIKLD VIGGDGDDVV LANASRIHYD GGAGTNTVSY AALGRQDSIT VSADGERFNV
961 RKQLNNANVY REGVATQTTA YGKRTENVQY RHVELARVGQ LVEVDTLEHV QHIIGGAGND
1021 SITGNAHDNF LAGGSGDDRL DGGAGNDTLV GGEGQNTVIG GAGDDVFLQD LGVWSNQLDG
1081 GAGVDTVKYN VHQPSEERLE RMGDTGIHAD LQKGTVEKWP ALNLFSVDHV KNIENLHGSR
1141 LNDRIAGDDQ DNELWGHDGN DTIRGRGGDD ILRGGLGLDT LYGEDGNDIF LQDDETVSDD
1201 IDGGAGLDTV DYSAMIHPGR IVAPHEYGFG IEADLSREWV RKASALGVDY YDNVRNVENV
1261 IGTSMKDVLI GDAQANTLMG QGGDDTVRGG DGDDLLFGGD GNDMLYGDAG NDTLYGGLGD
1321 DTLEGGAGND WFGQTQAREH DVLRGGDGVD TVDYSQTGAH AGIAAGRIGL GILADLGAGR
1381 VDKLGEAGSS AYDTVSGIEN VVGTELADRI TGDAQANVLR GAGGADVLAG GEGDDVLLGG
1441 DGDDQLSGDA GRDRLYGEAG DDWFFQDAAN AGNLLDGGDG RDTVDFSGPG RGLDAGAKGV
1501 FLSLGKGFAS LMDEPETSNV LRNIENAVGS ARDDVLIGDA GANVLNGLAG NDVLSGGAGD
1561 DVLLGDEGSD LLSGDAGNDD LFGGQGDDTY LFGVGYGHDT IYESGGGHDT IRINAGADQL
1621 WFARQGNDLE IRILGTDDAL TVHDWYRDAD HRVEIIHAAN QAVDQAGIEK LVEAMAQYPD
1681 PGAAAAAPPA ARVPDTLMQS LAVNWR
SEQ ID NO. 3 - mutated CyaA polypeptide sequence fragment 373-1706 bearing A680P and V695P mutations: 
373 SKFSPDVL ETVPASPGLR RPSLGAVERQ DSGYDSLDGV GSRSFSLGEV
421 SDMAAVEAAE LEMTRQVLHA GARQDDAEPG VSGASAHWGQ RALQGAQAVA AAQRLVHAIA
481 LMTQFGRAGS TNTPQEAASL SAAVFGLGEA SSAVAETVSG FFRGSSRWAG GFGVAGGAMA
541 LGGGIAAAVG AGMSLTDDAP AGQKAAAGAE IALQLTGGTV ELASSIALAL AAARGVTSGL
601 QVAGASAGAA AGALAAALSP MEIYGLVQQS HYADQLDKLA QESSAYGYEG DALLAQLYRD
661 KTAAEGAVAG VSAVLSTVGP AVSIAAAASV VGAPPAVVTS LLTGALNGIL RGVQQPIIEK
721 LANDYARKID ELGGPQAYFE KNLQARHEQL ANSDGLRKML ADLQAGWNAS SVIGVQTTEI
781 SKSALELAAI TGNADNLKSV DVFVDRFVQG ERVAGQPVVL DVAAGGIDIA SRKGERPALT
841 FITPLAAPGE EQRRRTKTGK SEFTTFVEIV GKQDRWRIRD GAADTTIDLA KVVSQLVDAN
901 GVLKHSIKLD VIGGDGDDVV LANASRIHYD GGAGTNTVSY AALGRQDSIT VSADGERFNV
961 RKQLNNANVY REGVATQTTA YGKRTENVQY RHVELARVGQ LVEVDTLEHV QHIIGGAGND
1021 SITGNAHDNF LAGGSGDDRL DGGAGNDTLV GGEGQNTVIG GAGDDVFLQD LGVWSNQLDG
1081 GAGVDTVKYN VHQPSEERLE RMGDTGIHAD LQKGTVEKWP ALNLFSVDHV KNIENLHGSR
1141 LNDRIAGDDQ DNELWGHDGN DTIRGRGGDD ILRGGLGLDT LYGEDGNDIF LQDDETVSDD
1201 IDGGAGLDTV DYSAMIHPGR IVAPHEYGFG IEADLSREWV RKASALGVDY YDNVRNVENV
1261 IGTSMKDVLI GDAQANTLMG QGGDDTVRGG DGDDLLFGGD GNDMLYGDAG NDTLYGGLGD
1321 DTLEGGAGND WFGQTQAREH DVLRGGDGVD TVDYSQTGAH AGIAAGRIGL GILADLGAGR
1381 VDKLGEAGSS AYDTVSGIEN VVGTELADRI TGDAQANVLR GAGGADVLAG GEGDDVLLGG
1441 DGDDQLSGDA GRDRLYGEAG DDWFFQDAAN AGNLLDGGDG RDTVDFSGPG RGLDAGAKGV
 
1501 FLSLGKGFAS LMDEPETSNV LRNIENAVGS ARDDVLIGDA GANVLNGLAG NDVLSGGAGD
1561 DVLLGDEGSD LLSGDAGNDD LFGGQGDDTY LFGVGYGHDT IYESGGGHDT IRINAGADQL
1621 WFARQGNDLE IRILGTDDAL TVHDWYRDAD HRVEIIHAAN QAVDQAGIEK LVEAMAQYPD
1681 PGAAAAAPPA ARVPDTLMQS LAVNWR
SEQ ID NO. 4 - mutated CyaA polypeptide sequence fragment 372-1706 bearing A680P and V695P mutations: 
372 RSKFSPDVL ETVPASPGLR RPSLGAVERQ DSGYDSLDGV GSRSFSLGEV
421 SDMAAVEAAE LEMTRQVLHA GARQDDAEPG VSGASAHWGQ RALQGAQAVA AAQRLVHAIA
481 LMTQFGRAGS TNTPQEAASL SAAVFGLGEA SSAVAETVSG FFRGSSRWAG GFGVAGGAMA
541 LGGGIAAAVG AGMSLTDDAP AGQKAAAGAE IALQLTGGTV ELASSIALAL AAARGVTSGL
601 QVAGASAGAA AGALAAALSP MEIYGLVQQS HYADQLDKLA QESSAYGYEG DALLAQLYRD
661 KTAAEGAVAG VSAVLSTVGP AVSIAAAASV VGAPPAVVTS LLTGALNGIL RGVQQPIIEK
721 LANDYARKID ELGGPQAYFE KNLQARHEQL ANSDGLRKML ADLQAGWNAS SVIGVQTTEI
781 SKSALELAAI TGNADNLKSV DVFVDRFVQG ERVAGQPVVL DVAAGGIDIA SRKGERPALT
841 FITPLAAPGE EQRRRTKTGK SEFTTFVEIV GKQDRWRIRD GAADTTIDLA KVVSQLVDAN
901 GVLKHSIKLD VIGGDGDDVV LANASRIHYD GGAGTNTVSY AALGRQDSIT VSADGERFNV
961 RKQLNNANVY REGVATQTTA YGKRTENVQY RHVELARVGQ LVEVDTLEHV QHIIGGAGND
1021 SITGNAHDNF LAGGSGDDRL DGGAGNDTLV GGEGQNTVIG GAGDDVFLQD LGVWSNQLDG
1081 GAGVDTVKYN VHQPSEERLE RMGDTGIHAD LQKGTVEKWP ALNLFSVDHV KNIENLHGSR
1141 LNDRIAGDDQ DNELWGHDGN DTIRGRGGDD ILRGGLGLDT LYGEDGNDIF LQDDETVSDD
1201 IDGGAGLDTV DYSAMIHPGR IVAPHEYGFG IEADLSREWV RKASALGVDY YDNVRNVENV
1261 IGTSMKDVLI GDAQANTLMG QGGDDTVRGG DGDDLLFGGD GNDMLYGDAG NDTLYGGLGD
1321 DTLEGGAGND WFGQTQAREH DVLRGGDGVD TVDYSQTGAH AGIAAGRIGL GILADLGAGR
1381 VDKLGEAGSS AYDTVSGIEN VVGTELADRI TGDAQANVLR GAGGADVLAG GEGDDVLLGG
1441 DGDDQLSGDA GRDRLYGEAG DDWFFQDAAN AGNLLDGGDG RDTVDFSGPG RGLDAGAKGV
1501 FLSLGKGFAS LMDEPETSNV LRNIENAVGS ARDDVLIGDA GANVLNGLAG NDVLSGGAGD
1561 DVLLGDEGSD LLSGDAGNDD LFGGQGDDTY LFGVGYGHDT IYESGGGHDT IRINAGADQL
1621 WFARQGNDLE IRILGTDDAL TVHDWYRDAD HRVEIIHAAN QAVDQAGIEK LVEAMAQYPD
1681 PGAAAAAPPA ARVPDTLMQS LAVNWR
SEQ ID NO. 5 - mutated CyaA polypeptide sequence 1-1706 bearing A680P and V695P mutations: 
1 MQQSHQAGYA NAADRESGIP AAVLDGIKAV AKEKNATLMF RLVNPHSTSL IAEGVATKGL
61 GVHAKSSDWG LQAGYIPVNP NLSKLFGRAP EVIARADNDV NSSLAHGHTA VDLTLSKERL
121 DYLRQAGLVT GMADGVVASN HAGYEQFEFR VKETSDGRYA VQYRRKGGDD FEAVKVIGNA
181 AGIPLTADID MFAIMPHLSN FRDSARSSVT SGDSVTDYLA RTRRAASEAT GGLDRERIDL
241 LWKIARAGAR SAVGTEARRQ FRYDGDMNIG VITDFELEVR NALNRRAHAV GAQDVVQHGT
301 EQNNPFPEAD EKIFVVSATG ESQMLTRGQL KEYIGQQRGE GYVFYENRAY GVAGKSLFDD
361 GLGAAPGVPS GRSKFSPDVL ETVPASPGLR RPSLGAVERQ DSGYDSLDGV GSRSFSLGEV
421 SDMAAVEAAE LEMTRQVLHA GARQDDAEPG VSGASAHWGQ RALQGAQAVA AAQRLVHAIA
481 LMTQFGRAGS TNTPQEAASL SAAVFGLGEA SSAVAETVSG FFRGSSRWAG GFGVAGGAMA
541 LGGGIAAAVG AGMSLTDDAP AGQKAAAGAE IALQLTGGTV ELASSIALAL AAARGVTSGL
601 QVAGASAGAA AGALAAALSP MEIYGLVQQS HYADQLDKLA QESSAYGYEG DALLAQLYRD
661 KTAAEGAVAG VSAVLSTVGP AVSIAAAASV VGAPPAVVTS LLTGALNGIL RGVQQPIIEK
721 LANDYARKID ELGGPQAYFE KNLQARHEQL ANSDGLRKML ADLQAGWNAS SVIGVQTTEI
781 SKSALELAAI TGNADNLKSV DVFVDRFVQG ERVAGQPVVL DVAAGGIDIA SRKGERPALT
841 FITPLAAPGE EQRRRTKTGK SEFTTFVEIV GKQDRWRIRD GAADTTIDLA KVVSQLVDAN
901 GVLKHSIKLD VIGGDGDDVV LANASRIHYD GGAGTNTVSY AALGRQDSIT VSADGERFNV
961 RKQLNNANVY REGVATQTTA YGKRTENVQY RHVELARVGQ LVEVDTLEHV QHIIGGAGND
1021 SITGNAHDNF LAGGSGDDRL DGGAGNDTLV GGEGQNTVIG GAGDDVFLQD LGVWSNQLDG
1081 GAGVDTVKYN VHQPSEERLE RMGDTGIHAD LQKGTVEKWP ALNLFSVDHV KNIENLHGSR
1141 LNDRIAGDDQ DNELWGHDGN DTIRGRGGDD ILRGGLGLDT LYGEDGNDIF LQDDETVSDD
 
1201 IDGGAGLDTV DYSAMIHPGR IVAPHEYGFG IEADLSREWV RKASALGVDY YDNVRNVENV
1261 IGTSMKDVLI GDAQANTLMG QGGDDTVRGG DGDDLLFGGD GNDMLYGDAG NDTLYGGLGD
1321 DTLEGGAGND WFGQTQAREH DVLRGGDGVD TVDYSQTGAH AGIAAGRIGL GILADLGAGR
1381 VDKLGEAGSS AYDTVSGIEN VVGTELADRI TGDAQANVLR GAGGADVLAG GEGDDVLLGG
1441 DGDDQLSGDA GRDRLYGEAG DDWFFQDAAN AGNLLDGGDG RDTVDFSGPG RGLDAGAKGV
1501 FLSLGKGFAS LMDEPETSNV LRNIENAVGS ARDDVLIGDA GANVLNGLAG NDVLSGGAGD
1561 DVLLGDEGSD LLSGDAGNDD LFGGQGDDTY LFGVGYGHDT IYESGGGHDT IRINAGADQL
1621 WFARQGNDLE IRILGTDDAL TVHDWYRDAD HRVEIIHAAN QAVDQAGIEK LVEAMAQYPD
1681 PGAAAAAPPA ARVPDTLMQS LAVNWR
SEQ ID NO. 6 - mutated CyaA polypeptide sequence 1-1706 bearing A680P and V695P mutations, and GlySer dipeptide insert between the amino acids in positions 188-189:

1 MQQSHQAGYA NAADRESGIP AAVLDGIKAV AKEKNATLMF RLVNPHSTSL IAEGVATKGL
61 GVHAKSSDWG LQAGYIPVNP NLSKLFGRAP EVIARADNDV NSSLAHGHTA VDLTLSKERL
121 DYLRQAGLVT GMADGVVASN HAGYEQFEFR VKETSDGRYA VQYRRKGGDD FEAVKVIGNA
181 AGIPLTADGSID MFAIMPHLSN FRDSARSSVT SGDSVTDYLA RTRRAASEAT GGLDRERIDL
243 LWKIARAGAR SAVGTEARRQ FRYDGDMNIG VITDFELEVR NALNRRAHAV GAQDVVQHGT
303 EQNNPFPEAD EKIFVVSATG ESQMLTRGQL KEYIGQQRGE GYVFYENRAY GVAGKSLFDD
363 GLGAAPGVPS GRSKFSPDVL ETVPASPGLR RPSLGAVERQ DSGYDSLDGV GSRSFSLGEV
423 SDMAAVEAAE LEMTRQVLHA GARQDDAEPG VSGASAHWGQ RALQGAQAVA AAQRLVHAIA
483 LMTQFGRAGS TNTPQEAASL SAAVFGLGEA SSAVAETVSG FFRGSSRWAG GFGVAGGAMA
543 LGGGIAAAVG AGMSLTDDAP AGQKAAAGAE IALQLTGGTV ELASSIALAL AAARGVTSGL
603 QVAGASAGAA AGALAAALSP MEIYGLVQQS HYADQLDKLA QESSAYGYEG DALLAQLYRD
663 KTAAEGAVAG VSAVLSTVGP AVSIAAAASV VGAPPAVVTS LLTGALNGIL RGVQQPIIEK
723 LANDYARKID ELGGPQAYFE KNLQARHEQL ANSDGLRKML ADLQAGWNAS SVIGVQTTEI
783 SKSALELAAI TGNADNLKSV DVFVDRFVQG ERVAGQPVVL DVAAGGIDIA SRKGERPALT
843 FITPLAAPGE EQRRRTKTGK SEFTTFVEIV GKQDRWRIRD GAADTTIDLA KVVSQLVDAN
903 GVLKHSIKLD VIGGDGDDVV LANASRIHYD GGAGTNTVSY AALGRQDSIT VSADGERFNV
963 RKQLNNANVY REGVATQTTA YGKRTENVQY RHVELARVGQ LVEVDTLEHV QHIIGGAGND
1023 SITGNAHDNF LAGGSGDDRL DGGAGNDTLV GGEGQNTVIG GAGDDVFLQD LGVWSNQLDG
1083 GAGVDTVKYN VHQPSEERLE RMGDTGIHAD LQKGTVEKWP ALNLFSVDHV KNIENLHGSR
1143 LNDRIAGDDQ DNELWGHDGN DTIRGRGGDD ILRGGLGLDT LYGEDGNDIF LQDDETVSDD
1203 IDGGAGLDTV DYSAMIHPGR IVAPHEYGFG IEADLSREWV RKASALGVDY YDNVRNVENV
1263 IGTSMKDVLI GDAQANTLMG QGGDDTVRGG DGDDLLFGGD GNDMLYGDAG NDTLYGGLGD
1323 DTLEGGAGND WFGQTQAREH DVLRGGDGVD TVDYSQTGAH AGIAAGRIGL GILADLGAGR
1383 VDKLGEAGSS AYDTVSGIEN VVGTELADRI TGDAQANVLR GAGGADVLAG GEGDDVLLGG
1443 DGDDQLSGDA GRDRLYGEAG DDWFFQDAAN AGNLLDGGDG RDTVDFSGPG RGLDAGAKGV
1503 FLSLGKGFAS LMDEPETSNV LRNIENAVGS ARDDVLIGDA GANVLNGLAG NDVLSGGAGD
1563 DVLLGDEGSD LLSGDAGNDD LFGGQGDDTY LFGVGYGHDT IYESGGGHDT IRINAGADQL
1623 WFARQGNDLE IRILGTDDAL TVHDWYRDAD HRVEIIHAAN QAVDQAGIEK LVEAMAQYPD
1683 PGAAAAAPPA ARVPDTLMQS LAVNWR

In a second aspect, the present invention relates to a chimeric protein comprising the polypeptide antigen as defined above that is fused to a heterologous polypeptide.
Thus, the mutant polypeptide antigens of the invention may be further combined with non-CyaA molecules, thereby giving rise to chimeric proteins, wherein said polypeptide antigens or chimeric proteins have a preventive vaccinal and/or therapeutic effect when administered to a human.

The invention thus also relates to a chimeric protein (a derivative of the polypeptide antigen of the invention) which comprises or consists of a mutant CyaA-derived polypeptide antigen according to the invention recombined to one or more molecules, in particular to one or more molecules suitable for eliciting an immune response, thus constituting a recombinant or fusion or chimeric protein. The invention also relates to chimeric proteins obtained by chemically grafting said molecule(s) to the polypeptide antigens.

In a third aspect, the invention relates to a polynucleotide encoding the polypeptide antigen of the present invention or a chimeric protein of the present invention, and optionally encoding also the protein toxin acyltransferase *cyaC*.

In a fourth aspect, the present invention relates to a vector comprising a polynucleotide as described above. Preferably, the plasmid vector further comprises an origin of replication, such as the high copy replication origin colE1, a marker gene for selection for the presence of the vector in a cell, such as an antibiotic resistance gene, a gene for a transcriptional repressor, such as the LacI repressor, enabling inducible and thus regulated expression of the polynucleotides (genes) encoding the polypeptide antigen of the present invention or a chimeric protein of the present invention and optionally the gene for the protein toxin acyltransferase *cyaC*, such as through transcription under the control of LacI-regulated *lacZ* gene promoters.

In a fifth aspect, the present invention relates to a host cell genetically engineered with the polynucleotide or with the vector described previously, such as the *Escherichia coli B* strain BL21 or the *Escherichia coli* K12 strain XL-1 Blue or NM554 in which high level production of the polypeptide antigen of the present invention or a chimeric protein of the present invention is achieved by induction in the presence of an inducer compound, such as isopropyl β-D-1-thiogalactopyranoside.

In a sixth aspect, the polypeptide antigens and chimeric proteins according to the invention are suitable for use in prophylactic treatment and especially in vaccination and in therapy including in immunotherapy, in particular for eliciting an immune response in a subject, preferably a human subject.

In a seventh aspect, the present invention relates to a vaccine against a disease caused by *Bordetella pertussis,* in particular against pertussis or whooping cough, comprising the mutated polypeptide antigen derived from CyaA according to the present invention or the chimeric protein of the present invention, at least one pharmaceutically acceptable auxiliary substance, and optionally an adjuvant.

The auxiliary substances include pharmaceutically acceptable solvents, such as water, alcohols, lactose, surfactants and emulsifiers, such as polysorbates (e.g. Tween 80), pH-adjusting components, inorganic salts, etc.

Examples of suitable adjuvants comprise aluminium salts such as hydrated aluminium hydroxide or aluminium phosphate, lipopolysaccharide derived monophosphoryl lipid A, squalene-based MF59 adjuvant, chitosan etc.

The vaccine may preferably be in the form of suspension, more preferably aqueous suspension. The routes of administration may include injection, such as intramuscular injection, subcutaneous injection, intradermal injection and sublingual or intranasal administration.

### Brief description of the drawings

**Figure 1** depicts the scheme of the structure of the CyaA molecule. CyaA is a 1706-residue-long polypeptide that consists of an adenylate cyclase (AC) enzyme domain and a pore-forming RTX hemolysin.
   The RTX hemolysin moiety (Hly) contains a hydrophobic pore-forming domain comprising residues 500 to 700; an acylated domain between residues 800 and 1000, where the posttranslational acylation at two lysine residues (K⁸⁶⁰ and K⁹⁸³) occurs; a typical calcium-loaded receptor-binding RTX domain and C-terminal secretion signal. The zoom-out shows the prediction of secondary structures of the segments of the pore-forming domain. The putative transmembrane α-helix, predicted by the method of Eisenberg (Eisenberg et al. 1984, . J Mol Biol 179, pp. 125-142) is shown with indication of the A680 and V695 residues, the substitution of which yields specifically a reduced pore-forming activity.
**Figure 2** documents the toxin activities CyaA-A680P+V695P of Example 1 on two types of target cells, the sheep erythrocytes (A) and the J774A.1 murine macrophage cells (B), respectively. The determined relative cell-associated AC domain activity (Binding), translocation capacity (Invasive AC) and hemolytic activity (Hemolysis) of CyaA wild-type and of CyaA-A680+V695P are shown next to each other for comparison.
**Figure 3** shows the overall membrane activity of 1 nM CyaA and of 1 nM CyaA-A680P+V695P of Example 1 on artifial planar lipid bilayer membranes. The recordings of conductance measured across asolectin/n-decane membranes with applied membrane potential of -50 mV were acquired at 25°C.

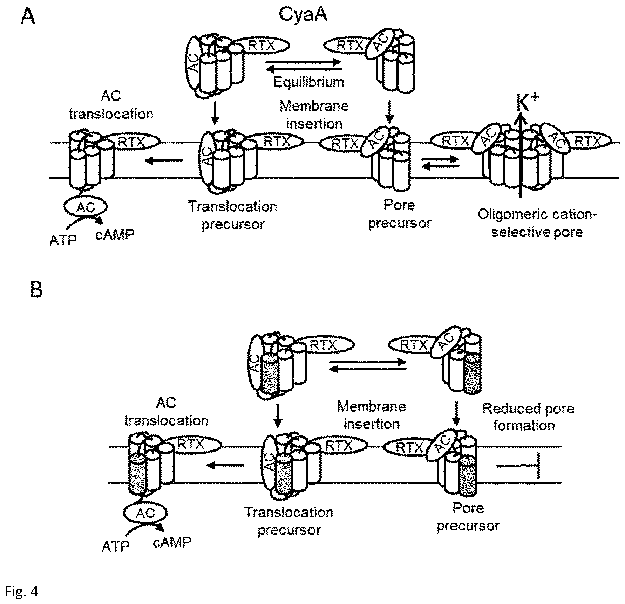

**Figure 4** shows a schematic model of the interaction of CyaA with target membrane. (A) In solution, two conformational isomers of CyaA exist in equilibrium and upon membrane insertion yield either a translocation precursor, competent for subsequent translocation of the AC domain across target membrane, or a pore precursor, competent for K⁺ efflux and involved in formation of oligomeric CyaA pores (Masin et al. 2015 Pathog Dis 73(8): ftv075). **(B)** Proposed model for the membrane interaction of CyaA-A680P+V695P. The proline substitutions in the α-helix₆₇₈₋₆₉₈ of CyaA (marked in gray) selectively alter pore structure and reduce the propensity of formation and stability of the CyaA pore without impacting on AC membrane translocation.

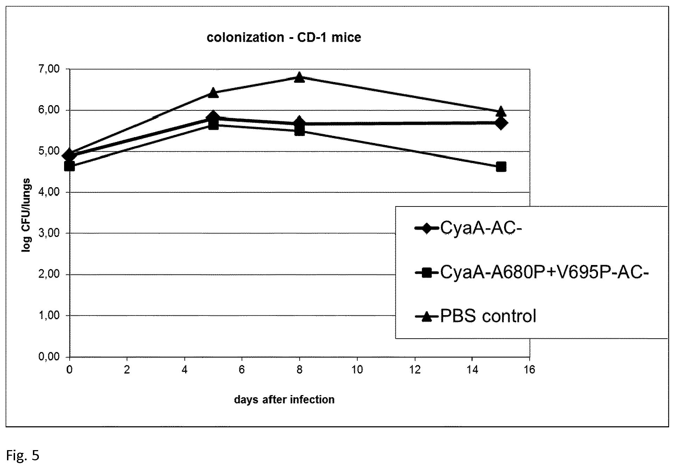

**Figure 5** **shows protection against intranasal challenge by *B. pertussis* that results from vaccination of mice with a toxoid form of CyaA-A680P+V695P of Example 1.** CD-1 mice were immunized intraperitoneally twice at a 3-week interval with 30 µg of the CyaA-AC⁻ or CyaA-A680P+V695P-AC⁻ toxoids adsorbed on aluminium hydroxide. Two weeks after the second immunization, the mice were intranasally infected with 1.5 x 10⁵ CFU of *B. pertussis* CIP 81.32 (Tohama I) and four mice per group were sacrificed on days 0, 5, 8, and 15, respectively. Homogenates of aseptically removed lungs were prepared and their serial dilutions were plated on BG agar. The numbers of CFU per lung were counted after 5 days of culture. The graph shows that a toxoid form of CyaA-A680P+V695P of Example 1, when used as immunogen for vaccination of mice, does confer on such vaccinated mice the same level of protection against intranasal infection as does the toxoid of CyaA wild-type.

### Examples

### Example 1. Construction, production and purification of CyaA-A680P+V695P.

Towards generating the mutant CyaA polypeptide antigen, the plasmid pCACT3 was used for co-expression of *cyaC* and *cyaA* genes allowing production of recombinant CyaC-activated CyaA in *Escherichia coli* (Betsou F. et al., 1993, Infection and Immunity 61, pp. 3583-3589). To introduce residue substitutions into CyaA, oligonucleotide-directed mutagenesis was performed to construct the pCACT3-derived plasmid (pCACT3-A680P+V695P) for expression of CyaA-A680P+V695P. Briefly, the primer 5'-C CTG AGC ACG GTG GGG **CCG** GCC GTG TCG ATC GCC GCG G-3' (SEQ ID NO. 7) was used for introduction of the Pro codon (bold underlined) at position 680 and the primer 5'-G ATC GCC GCG GCG GCT AGC GTG GTA GGG GCC CCG **CCG** GCG GTG GTC ACT TCC-3' (SEQ ID NO. 8) was used for introduction of the Pro codon (bold underlined) at position 695 of the *cyaA* gene, respectively, to yield pCACT3-A680P+V695P. Clones producing CyaA with introduced mutations were identified by restriction analysis of plasmids with enzymes *Fse* I (A680P substitution) and *Nhe* I (V695P substitution) on the introduction of the intended mutations and absence of unintended mutations was verified by DNA sequencing of the plasmid constructs. Intact CyaA and its mutant CyaA-A680P+V695P were next produced in *E. coli* XL1-Blue (Stratagene) bacteria that were transformed with appropriate pCACT3-derived plasmid construct. Recombinant CyaA synthesis was induced in exponential 250 ml cultures grown in Luria-Bertani broth using 1mM isopropyl 1-thio-β-D-galactopyranoside for 4 h. Harvested bacterial cells were disrupted by ultrasound, washed inclusion bodies were extracted with 8 M urea in 50 mM Tris-HCl (pH 8.0) and 0.2 mM CaCl₂, and the proteins were further purified by ionexchange chromatography on DEAE-Sepharose followed by hydrophobic chromatography on Phenyl-Sepharose se described earlier (Karimova et al. 1998 Proc. Natl. Acad. Sci U S A. 95, pp. 12532-12537). This yielded 5 milligrams of purified CyaA or CyaA-A680P+V695P per 1 L of bacterial culture, enabling characterization of biological and immunological properties of purified CyaA-A680P+V695P and their comparison to those of CyaA wild type.

### Example 2. CyaA-A680P+V695P is intact for cell binding and cell invasive activities but is impaired in hemolytic activities towards sheep erythrocytes.

The biological activities of CyaA-A680P+V695P produced as described under Example 1 were assessed using sheep erythrocytes. Towards this aim, the AC enzyme activities of purified CyaA and CyaA-A680P+V690P were measured in the presence of 1 µM calmodulin as previously described Ladant et al. 1988, J Biol Chem. 263, pp. 2612-2618. One unit of AC activity corresponds to 1 µmol of cAMP formed per min at 30°C, pH 8.0 and the measured activities values were used for adjusting CyaA and CyaA-A680P+V695P concentrations in the assays for toxin activities on target cells. Hemolytic activity of CyaA and CyaA-A680P+V695P was measured by determining the amount of hemoglobin liberated form sheep erythrocytes over time upon incubation with washed sheep erythrocytes (5×10⁸/ml), as previously described (Bellalou et al. 1990 Infect Immun 58(10), pp. 3242-3247). Cell invasive AC activity was measured as described previously (Masin et al. 2016 Sci. Rep. 6(1):29137 doi: 10.1038/srep29137), by determining the AC protected against externally added trypsin upon internalization into sheep erythrocytes. Erythrocyte binding of the toxins was determined as described in detail previously (Masin et al. 2016 Sci. Rep. 6(1):29137 doi: 10.1038/srep29137). Briefly, sheep erythrocytes (5x10⁸/ml) were incubated at 37°C with 1 µg/ml of intact CyaA or its A680+V695P mutant variant and after 30 min, aliquots were taken for determinations of the cell-associated AC activity (Binding) and of the AC activity internalized into erythrocytes and protected against digestion by externally added trypsin (Invasive AC). For determination of pore-forming (hemolytic activity), sheep erythrocytes (5x10⁸/ml) were incubated at 37°C in the presence of 10 µg/ml of intact CyaA or its mutant variants. Hemolytic activity was measured after 4 hours as the amount of released hemoglobin was determined by photometric measurement (A₅₄₁ₙₘ). As documented in Fig. 2A, the measurement results showed that CyaA-A680P+V695P exhibits the same specific capacity to bind sheep erythrocytes and to deliver into them the AC domain as does CyaA. At the same time, however, CyaA-A680P+V695P possesses a reduced capacity to form the cell-permeabilizing pores and exhibits a five-fold reduced specific capacity to provoke colloid-osmotic erythrocyte lysis (hemolysis). CyaA-A680P+V695P is thus selectively detoxified for the cytolytic capacity on mammalian cells.

**Example 3. CyaA-A680P+V695P is fully active for binding and penetration of J774A.1 murine macrophage cells.** To corroborate the observation presented under Example 2, the biological activities of CyaA and CyaA-A680P+V695P towards the murine macrophage cells expressing the CR3 receptor were compared. CyaA and CyaA-A680P+V695P were produced and purified under Example 1 and were incubated with murine J774A.1 macrophages (ATCC, number TIB-67). The target cells were grown at 37°C in a humidified air/CO₂ (19:1) atmosphere in RPMI medium supplemented with 10% (v/v) heat-inactivated fetal bovine serum, penicillin (100 i.u./ml), streptomycin (100 µg/ml) and amphotericin B (250 ng/ml). Prior to assays, RPMI was replaced with D-MEM medium (1.9 mM Ca²⁺) without FCS and the cells were allowed to rest in D-MEM for 1 h at 37°C in a humidified 5% CO₂ atmosphere (Basler et al. 2006 Infect Immun 74, pp. 2207-2214). J774A.1 cells (10⁶) were next incubated in D-MEM with 1 µg/ml of CyaA or CyaA-A680P+V695P for 30 min at 4°C, prior to removal of unbound toxin by three washes in D-MEM. After the transfer to the fresh tube, the cells were lyzed with 0.1% Triton X-100 for determination of cell-bound AC enzyme activity. For intracellular cAMP assays, 2x10⁵ J774A.1 cells were incubated at 37°C with CyaA (100, 50 25 and 12.5 ng/ml) for 30 min in D-MEM, the reaction was stopped by addition of 0.2% Tween-20 in 100 mM HCl, samples were boiled for 15 min at 100°C, neutralized by addition of 150 mM unbuffered imidazole and cAMP was measured by a competitive immunoassay (Masin et al. 2016 Sci. Rep. 6(1):29137 doi: 10.1038/srep29137). Activity of CyaA was taken as 100% and CyaA-A680P+V695P activities are expressed as percentages of intact CyaA activity. As documented in Fig. 2B, within the limits of the error of the experimental results, CyaA-A680P+V695P possesses the same macrophage cell-binding and macrophage penetrating (cAMP-elevating) activity as CyaA. Given that binding and penetration of J774A.1 cells depends on the capacity of CyaA to specifically bind the the complement receptor 3 (CR3) of myeloid cells, known also as the α_{M}β₂ integrin CD11b/CD18,or Mac-1 (Guermonprez, et al. 2001, J Exp Med 193, pp. 1035-1044; Osicka et al. 2015 Elife 4: e10766), these results revealed that the RTX hemolysin (Hly) moiety of CyaA-A680P+V695P folds correctly and possesses the conformational epitopes recognized by CyaA-neutralizing antibodies (Wang. et al. 2017, Biochemistry 56, pp. 1324-1336).

### Example 4. CyaA-A680P+V695P exhibits importantly reduced pore-forming capacity.

As outlined under Example 2, CyaA-A680P+V695P exhibited a 5-fold reduced capacity to provoke lysis of sheep erythrocytes. To corroborate that this was due to the reduced specific capacity of CyaA-A680P+V695P to form the cell-permeabilizing hemolytic pores, the specific pore-forming capacity of CyaA-A680P+V695P of Example 1, was measured on planar lipid bilayers. The black lipid membrane measurements were performed using Teflon cells separated by a diaphragm with a circular hole (diameter 0.5 mm) bearing the membrane, as described in detail previously (Masin et al. 2013 Infect. Immun. 81, pp. 4571-4582; Masin et al. 2016 Sci. Rep. 6(1):29137 doi: 10.1038/srep29137). This procedure measures the capacity of CyaA to insert into black lipid membrane bilayers and form ion-conducting pores that give rise to increasing conductance across the lipid bilayer. Black lipid bilayer membrane was formed by painting of soybean lecithin in n-decane-butanol (9:1, vol/vol) over the circular hole in the diaphragm of the Teflon cell. Both compartments of which contained 150 mM KCl, 10 mM Tris, and 2 mM CaCl₂ (pH 7.4), the temperature was 25°C. CyaA and CyaA-A680P+V695P purified as described under Example 1, were diluted in 8 M urea, 50 mM Tris-HCl (pH 8.0) and added to 1 nM concentration into the grounded cis compartment of the Teflon cell with applied positive potential. The membrane current was measured by Ag/AgCl electrodes (Theta) with salt bridges (applied voltage, -50 mV), amplified by LCA-200-100G amplifier (Femto), and digitized by use of a KPCI-3108 card (Keithly). Recording of the current (pA) in time revealed that by difference to CyaA the CyaA-A680P+V695P mutant was unable to produce the typical increase of membrane conductance over time. This shows that that capacity of the CyaA-A680P+V695P mutant to produce ion-conducting pores across lipid bilayer membranes is severely compromised due a devastating effect of the combination of the introduced A680P and V695P amino acid substitutions on the structure of the pore. This explains the 5-fold reduced capacity of CyaA-A680P+V695P to provoke lysis of sheep erythrocytes in comparison to the specific cytolytic activity of CyaA.

### Example 5. A toxoid of CyaA-A680P+V695P-AC⁻ possesses the same protective antigen capacity against intranasal infection by Bordetella pertussis as the toxoid of CyaA.

The protective antigen capacity of CyaA-A680P+V695P of Example 1 was compared to that of CyaA by using the polypeptide antigens as immunogens for vaccination of mice. Vaccinated mice were then intranasally challenged by a suspension of virulent of *Bordetella pertussis* bacteria. Towards this aim, toxoids of CyaA and CyaA-A680P+V695P were first constructed by introducing dipetide inserts into the AC domains of the polypeptide antigens to ablate their enzymatic AC activity. Double stranded synthetic oligonucleotides of the sequence 5'-GGATCC-3' encoding a GlySer dipetide were inserted into the EcoR V restriction sites located between codons 188 and 189 of the *cyaA* gene alleles encoding the CyaA and CyaA-A680P+V695P proteins on the pCACT3-derived vectors. The thus obtained vectors were used for production of the CyaA-AC⁻ and CyaA-A680P+V695P-AC⁻ toxoids that have the ATP binding sites of the adenylate cyclase enzyme domain disrupted by the 188GlySer189 dipetide insert and thus exhibit no detectable enzymatic activity (Ladant et al. 1992 J. Biol. Chem. 267, pp.2244-50). The resulting CyaA-AC⁻ and CyaA-A680P+V695P-AC⁻ toxoids were produced and purified as described under Example 1.

Protective immunity induced by the CyaA-AC⁻ and CyaA-A680P+V695P-AC⁻ toxoids was next determined by using them for vaccination of mice that were subsequently exposed to intranasal infection with suspensions of virulent *B. pertussis* strain CIP 81.32 (Tohama I), as previously described (Betsou et al., 1993, Infect. Immun. 61, pp. 3583-3589; Betsou et al., 1995, Infect. Immun. 63, pp. 3309-3315; Villarino Romero et al. 2013, Infect. Immun. 81, pp. 2761-2767.)

For mice immunization, 30 µg of CyaA-AC⁻ or antigen CyaA-A680P+V695P-AC⁻ in 180 µl of PBS were mixed with 20 µl of 2% aluminium hydroxide hydrate (A1577, Sigma-Aldrich) and left for 30 minutes on ice prior to injection. 17 female 4-week-old CD-1 mice per test group (Charles Rivers Genetic Models, Inc., Indianapolis, IN) were injected intraperitoneally twice at a 3-week interval, while control mice received only aluminium hydroxide adjuvant in PBS buffer.

Wild-type *B. pertussis* CIP 81.32 (Tohama I) bacteria were grown on Bordet-Gengou (BG) agar supplemented with 15% defibrinated sheep blood at 37°C for 72 h. For animal studies, subcultures of *B. pertussis* CIP 81.32 were performed in Stainer-Scholte medium {Stainer, 1970, 4324651} for 20 h at 37°C until the optical density reached 1.2 at 650 nm.

Two weeks after the second immunization with toxoids, the mice were intranasally infected with 1.5 x 10⁵ CFU of *B. pertussis* CIP 81.32 (Tohama I). 4 mice of each group were sacrificed on days 0, 5, 8, and 15, respectively and their lungs were aseptically removed and homogenized. Serial dilutions of lung homogenates were plated on BG agar and colony-forming units (CFU) were counted after 5 days of growth at at 37°C in a humified 5% CO₂ atmosphere.

For determination of protection against bacterial colonization, vaccinated BALB/c mice were challenged intranasally with 1.5 x 10⁵ CFU of *B. pertussis* CIP 81.32 on day 14 after the second immunization.

As documented in Fig. 5, in lungs of mice immunized by either of the two toxoids the *B. pertussis* bacteria proliferated on days 5 and 8 to ten-fold lower CFU levels than in the lungs of the infected naive control animals that received only PBS buffer. This shows that when used as immunogen, the toxoid CyaA-A680P+V695P-AC⁻ conferred on mice at least as high level of protection against infection by virulent *Bordetella pertussis* bacteria as the CyaA-AC⁻ toxoid. This demonstrates that the epitopes for toxin-neutralizing antibodies are preserved in CyaA-A680P+V695P to the same extent as in CyaA wild type.

## Claims

1. A polypeptide antigen comprising amino acid sequence of adenylate cyclase protein of *Bordetella pertussis* bearing the following mutations:
(i) substitution of the alanine residue in position 680 by a proline residue, and
(ii) substitution of the valine residue in position 695 by a proline residue,
or one or more fragments thereof, wherein said fragments comprise at least amino acids from position 374 to position 400, from position 500 to position 700, and from position 860 to position 1706.

2. The polypeptide antigen according to claim 1, wherein the protein contains amino acid sequence of adenylate cyclase protein bearing said mutations and having additionally:
(iii) deletion of any or all amino acids in positions 1 to 100-300, or in positions 1 to 100-200, or in positions 1 to 371, or in positions 1 to 372, or in positions 1 to 373, and/or
(iv) insertion of a dipeptide, preferably of a GlySer dipeptide, between amino acid residues in positions 188-189 of the amino acid sequence of adenylate cyclase protein.

3. The polypeptide antigen according to claim 1, comprising amino acid sequence of adenylate cyclase protein from position 374 to position 1706, bearing the following mutations:
(i) substitution of the alanine residue in position 680 by a proline residue, and
(ii) substitution of the valine residue in position 695 by a proline residue.

4. The polypeptide antigen according to claim 1, comprising the amino acid sequence selected from SEQ ID NO. 2, SEQ ID NO. 3, SEQ ID NO. 4.

5. The polypeptide antigen according to claim 1, comprising the amino acid sequence SEQ ID NO. 5 or SEQ ID NO. 6.

6. A chimeric protein comprising the polypeptide antigen according to any one of the preceding claims fused to at least one heterologous polypeptide, said heterologous polypeptide preferably being an antigen.

7. The chimeric protein according to claim 6, wherein the heterologous polypeptide is bound to the N-terminus of the peptide of any one of claims 1 to 5.

8. A polynucleotide comprising a nucleotide sequence encoding the polypeptide antigen according to any one of claims 1 to 5 or the chimeric protein according to claim 5 or 6, and optionally also a nucleotide sequence encoding protein toxin acyltransferase *cyaC*.

9. A vector comprising a polynucleotide according to claim 8.

10. A host cell comprising a polynucleotide according to claim 8, or a vector according to claim 9.

11. A vaccine against a disease caused by *Bordetella pertussis,* in particular against pertussis or whooping cough disease, comprising the polypeptide antigen according to any one of claims 1 to 5, or the chimeric protein according to claim 6 or 7, at least one pharmaceutically acceptable auxiliary substance, and optionally an adjuvant.

12. The polypeptide antigen according to any one of claims 1 to 5 or the chimeric protein according to claim 6 or 7 for use in a method of treatment and/or prevention of a disease caused by *Bordetella pertussis,* in particular against pertussis or whooping cough disease.

13. A method of treatment and/or prevention of a disease caused by *Bordetella pertussis,* in particular against pertussis or whooping cough, comprising the step of administering at least one polypeptide antigen according to any one of claims 1 to 5 or the chimeric protein according to claim 6 or 7 to a subject in need of such treatment and/or prevention.


SEQUENCE LISTING

<110>  Mikrobiologicky ustav AV CR, v. v. i.

<120>  Adenylate cyclase toxoid with reduced cytolytic activity

<130>  P1538

<160>  8

<170>  PatentIn version 3.5

<210>  1
<211>  1706
<212>  PRT
<213>  Bordetella pertussis

<400>  1

Met Gln Gln Ser His Gln Ala Gly Tyr Ala Asn Ala Ala Asp Arg Glu
1               5                   10                  15

Ser Gly Ile Pro Ala Ala Val Leu Asp Gly Ile Lys Ala Val Ala Lys
            20                  25                  30

Glu Lys Asn Ala Thr Leu Met Phe Arg Leu Val Asn Pro His Ser Thr
        35                  40                  45

Ser Leu Ile Ala Glu Gly Val Ala Thr Lys Gly Leu Gly Val His Ala
    50                  55                  60

Lys Ser Ser Asp Trp Gly Leu Gln Ala Gly Tyr Ile Pro Val Asn Pro
65                  70                  75                  80

Asn Leu Ser Lys Leu Phe Gly Arg Ala Pro Glu Val Ile Ala Arg Ala
                85                  90                  95

Asp Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu
        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala
145                 150                 155                 160

Val Gln Tyr Arg Arg Lys Gly Gly Asp Asp Phe Glu Ala Val Lys Val
                165                 170                 175

Ile Gly Asn Ala Ala Gly Ile Pro Leu Thr Ala Asp Ile Asp Met Phe
        180                 185                 190

Ala Ile Met Pro His Leu Ser Asn Phe Arg Asp Ser Ala Arg Ser Ser
    195                 200                 205

Val Thr Ser Gly Asp Ser Val Thr Asp Tyr Leu Ala Arg Thr Arg Arg
    210                 215                 220

Ala Ala Ser Glu Ala Thr Gly Gly Leu Asp Arg Glu Arg Ile Asp Leu
225                 230                 235                 240

Leu Trp Lys Ile Ala Arg Ala Gly Ala Arg Ser Ala Val Gly Thr Glu
                245                 250                 255

Ala Arg Arg Gln Phe Arg Tyr Asp Gly Asp Met Asn Ile Gly Val Ile
            260                 265                 270

Thr Asp Phe Glu Leu Glu Val Arg Asn Ala Leu Asn Arg Arg Ala His
        275                 280                 285

Ala Val Gly Ala Gln Asp Val Val Gln His Gly Thr Glu Gln Asn Asn
    290                 295                 300

Pro Phe Pro Glu Ala Asp Glu Lys Ile Phe Val Val Ser Ala Thr Gly
305                 310                 315                 320

Glu Ser Gln Met Leu Thr Arg Gly Gln Leu Lys Glu Tyr Ile Gly Gln
                325                 330                 335

Gln Arg Gly Glu Gly Tyr Val Phe Tyr Glu Asn Arg Ala Tyr Gly Val
            340                 345                 350

Ala Gly Lys Ser Leu Phe Asp Asp Gly Leu Gly Ala Ala Pro Gly Val
        355                 360                 365

Pro Ser Gly Arg Ser Lys Phe Ser Pro Asp Val Leu Glu Thr Val Pro
    370                 375                 380

Ala Ser Pro Gly Leu Arg Arg Pro Ser Leu Gly Ala Val Glu Arg Gln
385                 390                 395                 400

Asp Ser Gly Tyr Asp Ser Leu Asp Gly Val Gly Ser Arg Ser Phe Ser
                405                 410                 415

Leu Gly Glu Val Ser Asp Met Ala Ala Val Glu Ala Ala Glu Leu Glu
            420                 425                 430

Met Thr Arg Gln Val Leu His Ala Gly Ala Arg Gln Asp Asp Ala Glu
        435                 440                 445

Pro Gly Val Ser Gly Ala Ser Ala His Trp Gly Gln Arg Ala Leu Gln
    450                 455                 460

Gly Ala Gln Ala Val Ala Ala Ala Gln Arg Leu Val His Ala Ile Ala
465                 470                 475                 480

Leu Met Thr Gln Phe Gly Arg Ala Gly Ser Thr Asn Thr Pro Gln Glu
                485                 490                 495

Ala Ala Ser Leu Ser Ala Ala Val Phe Gly Leu Gly Glu Ala Ser Ser
            500                 505                 510

Ala Val Ala Glu Thr Val Ser Gly Phe Phe Arg Gly Ser Ser Arg Trp
        515                 520                 525

Ala Gly Gly Phe Gly Val Ala Gly Gly Ala Met Ala Leu Gly Gly Gly
    530                 535                 540

Ile Ala Ala Ala Val Gly Ala Gly Met Ser Leu Thr Asp Asp Ala Pro
545                 550                 555                 560

Ala Gly Gln Lys Ala Ala Ala Gly Ala Glu Ile Ala Leu Gln Leu Thr
                565                 570                 575

Gly Gly Thr Val Glu Leu Ala Ser Ser Ile Ala Leu Ala Leu Ala Ala
            580                 585                 590

Ala Arg Gly Val Thr Ser Gly Leu Gln Val Ala Gly Ala Ser Ala Gly
        595                 600                 605

Ala Ala Ala Gly Ala Leu Ala Ala Ala Leu Ser Pro Met Glu Ile Tyr
    610                 615                 620

Gly Leu Val Gln Gln Ser His Tyr Ala Asp Gln Leu Asp Lys Leu Ala
625                 630                 635                 640

Gln Glu Ser Ser Ala Tyr Gly Tyr Glu Gly Asp Ala Leu Leu Ala Gln
                645                 650                 655

Leu Tyr Arg Asp Lys Thr Ala Ala Glu Gly Ala Val Ala Gly Val Ser
            660                 665                 670

Ala Val Leu Ser Thr Val Gly Ala Ala Val Ser Ile Ala Ala Ala Ala
        675                 680                 685

Ser Val Val Gly Ala Pro Val Ala Val Val Thr Ser Leu Leu Thr Gly
    690                 695                 700

Ala Leu Asn Gly Ile Leu Arg Gly Val Gln Gln Pro Ile Ile Glu Lys
705                 710                 715                 720

Leu Ala Asn Asp Tyr Ala Arg Lys Ile Asp Glu Leu Gly Gly Pro Gln
                725                 730                 735

Ala Tyr Phe Glu Lys Asn Leu Gln Ala Arg His Glu Gln Leu Ala Asn
            740                 745                 750

Ser Asp Gly Leu Arg Lys Met Leu Ala Asp Leu Gln Ala Gly Trp Asn
        755                 760                 765

Ala Ser Ser Val Ile Gly Val Gln Thr Thr Glu Ile Ser Lys Ser Ala
    770                 775                 780

Leu Glu Leu Ala Ala Ile Thr Gly Asn Ala Asp Asn Leu Lys Ser Val
785                 790                 795                 800

Asp Val Phe Val Asp Arg Phe Val Gln Gly Glu Arg Val Ala Gly Gln
                805                 810                 815

Pro Val Val Leu Asp Val Ala Ala Gly Gly Ile Asp Ile Ala Ser Arg
            820                 825                 830

Lys Gly Glu Arg Pro Ala Leu Thr Phe Ile Thr Pro Leu Ala Ala Pro
        835                 840                 845

Gly Glu Glu Gln Arg Arg Arg Thr Lys Thr Gly Lys Ser Glu Phe Thr
    850                 855                 860

Thr Phe Val Glu Ile Val Gly Lys Gln Asp Arg Trp Arg Ile Arg Asp
865                 870                 875                 880

Gly Ala Ala Asp Thr Thr Ile Asp Leu Ala Lys Val Val Ser Gln Leu
                885                 890                 895

Val Asp Ala Asn Gly Val Leu Lys His Ser Ile Lys Leu Asp Val Ile
            900                 905                 910

Gly Gly Asp Gly Asp Asp Val Val Leu Ala Asn Ala Ser Arg Ile His
        915                 920                 925

Tyr Asp Gly Gly Ala Gly Thr Asn Thr Val Ser Tyr Ala Ala Leu Gly

930                 935                 940

Arg Gln Asp Ser Ile Thr Val Ser Ala Asp Gly Glu Arg Phe Asn Val
945                 950                 955                 960

Arg Lys Gln Leu Asn Asn Ala Asn Val Tyr Arg Glu Gly Val Ala Thr
                965                 970                 975

Gln Thr Thr Ala Tyr Gly Lys Arg Thr Glu Asn Val Gln Tyr Arg His
            980                 985                 990

Val Glu Leu Ala Arg Val Gly Gln  Leu Val Glu Val Asp  Thr Leu Glu
        995                 1000                 1005

His Val  Gln His Ile Ile Gly  Gly Ala Gly Asn Asp  Ser Ile Thr
    1010                 1015                 1020

Gly Asn  Ala His Asp Asn Phe  Leu Ala Gly Gly Ser  Gly Asp Asp
    1025                 1030                 1035

Arg Leu  Asp Gly Gly Ala Gly  Asn Asp Thr Leu Val  Gly Gly Glu
    1040                 1045                 1050

Gly Gln  Asn Thr Val Ile Gly  Gly Ala Gly Asp Asp  Val Phe Leu
    1055                 1060                 1065

Gln Asp  Leu Gly Val Trp Ser  Asn Gln Leu Asp Gly  Gly Ala Gly
    1070                 1075                 1080

Val Asp  Thr Val Lys Tyr Asn  Val His Gln Pro Ser  Glu Glu Arg
    1085                 1090                 1095

Leu Glu  Arg Met Gly Asp Thr  Gly Ile His Ala Asp  Leu Gln Lys
    1100                 1105                 1110

Gly Thr  Val Glu Lys Trp Pro  Ala Leu Asn Leu Phe  Ser Val Asp
    1115                 1120                 1125

His Val  Lys Asn Ile Glu Asn  Leu His Gly Ser Arg  Leu Asn Asp
    1130                 1135                 1140

Arg Ile  Ala Gly Asp Asp Gln  Asp Asn Glu Leu Trp  Gly His Asp
    1145                 1150                 1155

Gly Asn  Asp Thr Ile Arg Gly  Arg Gly Gly Asp Asp  Ile Leu Arg
    1160                 1165                 1170

Gly Gly Leu Gly Leu Asp Thr Leu Tyr Gly Glu Asp Gly Asn Asp
    1175                1180                1185

Ile Phe Leu Gln Asp Asp Glu Thr Val Ser Asp Asp Ile Asp Gly
    1190                1195                1200

Gly Ala Gly Leu Asp Thr Val Asp Tyr Ser Ala Met Ile His Pro
    1205                1210                1215

Gly Arg Ile Val Ala Pro His Glu Tyr Gly Phe Gly Ile Glu Ala
    1220                1225                1230

Asp Leu Ser Arg Glu Trp Val Arg Lys Ala Ser Ala Leu Gly Val
    1235                1240                1245

Asp Tyr Tyr Asp Asn Val Arg Asn Val Glu Asn Val Ile Gly Thr
    1250                1255                1260

Ser Met Lys Asp Val Leu Ile Gly Asp Ala Gln Ala Asn Thr Leu
    1265                1270                1275

Met Gly Gln Gly Gly Asp Asp Thr Val Arg Gly Gly Asp Gly Asp
    1280                1285                1290

Asp Leu Leu Phe Gly Gly Asp Gly Asn Asp Met Leu Tyr Gly Asp
    1295                1300                1305

Ala Gly Asn Asp Thr Leu Tyr Gly Gly Leu Gly Asp Asp Thr Leu
    1310                1315                1320

Glu Gly Gly Ala Gly Asn Asp Trp Phe Gly Gln Thr Gln Ala Arg
    1325                1330                1335

Glu His Asp Val Leu Arg Gly Gly Asp Gly Val Asp Thr Val Asp
    1340                1345                1350

Tyr Ser Gln Thr Gly Ala His Ala Gly Ile Ala Ala Gly Arg Ile
    1355                1360                1365

Gly Leu Gly Ile Leu Ala Asp Leu Gly Ala Gly Arg Val Asp Lys
    1370                1375                1380

Leu Gly Glu Ala Gly Ser Ser Ala Tyr Asp Thr Val Ser Gly Ile
    1385                1390                1395

Glu Asn Val Val Gly Thr Glu Leu Ala Asp Arg Ile Thr Gly Asp
    1400                1405                1410

Ala Gln Ala Asn Val Leu Arg Gly Ala Gly Gly Ala Asp Val Leu
    1415                1420                1425

Ala Gly Gly Glu Gly Asp Asp Val Leu Leu Gly Gly Asp Gly Asp
    1430                1435                1440

Asp Gln Leu Ser Gly Asp Ala Gly Arg Asp Arg Leu Tyr Gly Glu
    1445                1450                1455

Ala Gly Asp Asp Trp Phe Phe Gln Asp Ala Ala Asn Ala Gly Asn
    1460                1465                1470

Leu Leu Asp Gly Gly Asp Gly Arg Asp Thr Val Asp Phe Ser Gly
    1475                1480                1485

Pro Gly Arg Gly Leu Asp Ala Gly Ala Lys Gly Val Phe Leu Ser
    1490                1495                1500

Leu Gly Lys Gly Phe Ala Ser Leu Met Asp Glu Pro Glu Thr Ser
    1505                1510                1515

Asn Val Leu Arg Asn Ile Glu Asn Ala Val Gly Ser Ala Arg Asp
    1520                1525                1530

Asp Val Leu Ile Gly Asp Ala Gly Ala Asn Val Leu Asn Gly Leu
    1535                1540                1545

Ala Gly Asn Asp Val Leu Ser Gly Gly Ala Gly Asp Asp Val Leu
    1550                1555                1560

Leu Gly Asp Glu Gly Ser Asp Leu Leu Ser Gly Asp Ala Gly Asn
    1565                1570                1575

Asp Asp Leu Phe Gly Gly Gln Gly Asp Asp Thr Tyr Leu Phe Gly
    1580                1585                1590

Val Gly Tyr Gly His Asp Thr Ile Tyr Glu Ser Gly Gly Gly His
    1595                1600                1605

Asp Thr Ile Arg Ile Asn Ala Gly Ala Asp Gln Leu Trp Phe Ala
    1610                1615                1620

Arg Gln Gly Asn Asp Leu Glu Ile Arg Ile Leu Gly Thr Asp Asp
    1625                1630                1635

Ala Leu Thr Val His Asp Trp Tyr Arg Asp Ala Asp His Arg Val
    1640                1645                1650

Glu Ile Ile His Ala Ala Asn Gln Ala Val Asp Gln Ala Gly Ile
    1655                1660                1665

Glu Lys Leu Val Glu Ala Met Ala Gln Tyr Pro Asp Pro Gly Ala
    1670                1675                1680

Ala Ala Ala Ala Pro Pro Ala Ala Arg Val Pro Asp Thr Leu Met
    1685                1690                1695

Gln Ser Leu Ala Val Asn Trp Arg
    1700                1705

<210>  2
<211>  1333
<212>  PRT
<213>  artificial

<220>
<223>  CyaA fragment 373-1706, A680P V695P

<400>  2

Lys Phe Ser Pro Asp Val Leu Glu Thr Val Pro Ala Ser Pro Gly Leu
1               5                   10                  15

Arg Arg Pro Ser Leu Gly Ala Val Glu Arg Gln Asp Ser Gly Tyr Asp
            20                  25                  30

Ser Leu Asp Gly Val Gly Ser Arg Ser Phe Ser Leu Gly Glu Val Ser
        35                  40                  45

Asp Met Ala Ala Val Glu Ala Ala Glu Leu Glu Met Thr Arg Gln Val
    50                  55                  60

Leu His Ala Gly Ala Arg Gln Asp Asp Ala Glu Pro Gly Val Ser Gly
65                  70                  75                  80

Ala Ser Ala His Trp Gly Gln Arg Ala Leu Gln Gly Ala Gln Ala Val
                85                  90                  95

Ala Ala Ala Gln Arg Leu Val His Ala Ile Ala Leu Met Thr Gln Phe
            100                 105                 110

Gly Arg Ala Gly Ser Thr Asn Thr Pro Gln Glu Ala Ala Ser Leu Ser
        115                 120                 125

Ala Ala Val Phe Gly Leu Gly Glu Ala Ser Ser Ala Val Ala Glu Thr
    130                 135                 140

Val Ser Gly Phe Phe Arg Gly Ser Ser Arg Trp Ala Gly Gly Phe Gly
145                 150                 155                 160

Val Ala Gly Gly Ala Met Ala Leu Gly Gly Gly Ile Ala Ala Ala Val
                165                 170                 175

Gly Ala Gly Met Ser Leu Thr Asp Asp Ala Pro Ala Gly Gln Lys Ala
            180                 185                 190

Ala Ala Gly Ala Glu Ile Ala Leu Gln Leu Thr Gly Gly Thr Val Glu
        195                 200                 205

Leu Ala Ser Ser Ile Ala Leu Ala Leu Ala Ala Ala Arg Gly Val Thr
    210                 215                 220

Ser Gly Leu Gln Val Ala Gly Ala Ser Ala Gly Ala Ala Ala Gly Ala
225                 230                 235                 240

Leu Ala Ala Ala Leu Ser Pro Met Glu Ile Tyr Gly Leu Val Gln Gln
                245                 250                 255

Ser His Tyr Ala Asp Gln Leu Asp Lys Leu Ala Gln Glu Ser Ser Ala
            260                 265                 270

Tyr Gly Tyr Glu Gly Asp Ala Leu Leu Ala Gln Leu Tyr Arg Asp Lys
        275                 280                 285

Thr Ala Ala Glu Gly Ala Val Ala Gly Val Ser Ala Val Leu Ser Thr
    290                 295                 300

Val Gly Pro Ala Val Ser Ile Ala Ala Ala Ala Ser Val Val Gly Ala
305                 310                 315                 320

Pro Pro Ala Val Val Thr Ser Leu Leu Thr Gly Ala Leu Asn Gly Ile
                325                 330                 335

Leu Arg Gly Val Gln Gln Pro Ile Ile Glu Lys Leu Ala Asn Asp Tyr
            340                 345                 350

Ala Arg Lys Ile Asp Glu Leu Gly Gly Pro Gln Ala Tyr Phe Glu Lys
        355                 360                 365

Asn Leu Gln Ala Arg His Glu Gln Leu Ala Asn Ser Asp Gly Leu Arg
    370                 375                 380

Lys Met Leu Ala Asp Leu Gln Ala Gly Trp Asn Ala Ser Ser Val Ile
385                 390                 395                 400

Gly Val Gln Thr Thr Glu Ile Ser Lys Ser Ala Leu Glu Leu Ala Ala
                405                 410                 415

Ile Thr Gly Asn Ala Asp Asn Leu Lys Ser Val Asp Val Phe Val Asp
            420                 425                 430

Arg Phe Val Gln Gly Glu Arg Val Ala Gly Gln Pro Val Val Leu Asp
        435                 440                 445

Val Ala Ala Gly Gly Ile Asp Ile Ala Ser Arg Lys Gly Glu Arg Pro
    450                 455                 460

Ala Leu Thr Phe Ile Thr Pro Leu Ala Ala Pro Gly Glu Glu Gln Arg
465                 470                 475                 480

Arg Arg Thr Lys Thr Gly Lys Ser Glu Phe Thr Thr Phe Val Glu Ile
                485                 490                 495

Val Gly Lys Gln Asp Arg Trp Arg Ile Arg Asp Gly Ala Ala Asp Thr
            500                 505                 510

Thr Ile Asp Leu Ala Lys Val Val Ser Gln Leu Val Asp Ala Asn Gly
        515                 520                 525

Val Leu Lys His Ser Ile Lys Leu Asp Val Ile Gly Gly Asp Gly Asp
    530                 535                 540

Asp Val Val Leu Ala Asn Ala Ser Arg Ile His Tyr Asp Gly Gly Ala
545                 550                 555                 560

Gly Thr Asn Thr Val Ser Tyr Ala Ala Leu Gly Arg Gln Asp Ser Ile
                565                 570                 575

Thr Val Ser Ala Asp Gly Glu Arg Phe Asn Val Arg Lys Gln Leu Asn
            580                 585                 590

Asn Ala Asn Val Tyr Arg Glu Gly Val Ala Thr Gln Thr Thr Ala Tyr
        595                 600                 605

Gly Lys Arg Thr Glu Asn Val Gln Tyr Arg His Val Glu Leu Ala Arg
    610                 615                 620

Val Gly Gln Leu Val Glu Val Asp Thr Leu Glu His Val Gln His Ile
625                 630                 635                 640

Ile Gly Gly Ala Gly Asn Asp Ser Ile Thr Gly Asn Ala His Asp Asn

                645                 650                 655

Phe Leu Ala Gly Gly Ser Gly Asp Asp Arg Leu Asp Gly Gly Ala Gly
            660                 665                 670

Asn Asp Thr Leu Val Gly Gly Glu Gly Gln Asn Thr Val Ile Gly Gly
        675                 680                 685

Ala Gly Asp Asp Val Phe Leu Gln Asp Leu Gly Val Trp Ser Asn Gln
    690                 695                 700

Leu Asp Gly Gly Ala Gly Val Asp Thr Val Lys Tyr Asn Val His Gln
705                 710                 715                 720

Pro Ser Glu Glu Arg Leu Glu Arg Met Gly Asp Thr Gly Ile His Ala
                725                 730                 735

Asp Leu Gln Lys Gly Thr Val Glu Lys Trp Pro Ala Leu Asn Leu Phe
            740                 745                 750

Ser Val Asp His Val Lys Asn Ile Glu Asn Leu His Gly Ser Arg Leu
        755                 760                 765

Asn Asp Arg Ile Ala Gly Asp Asp Gln Asp Asn Glu Leu Trp Gly His
    770                 775                 780

Asp Gly Asn Asp Thr Ile Arg Gly Arg Gly Gly Asp Asp Ile Leu Arg
785                 790                 795                 800

Gly Gly Leu Gly Leu Asp Thr Leu Tyr Gly Glu Asp Gly Asn Asp Ile
                805                 810                 815

Phe Leu Gln Asp Asp Glu Thr Val Ser Asp Asp Ile Asp Gly Gly Ala
            820                 825                 830

Gly Leu Asp Thr Val Asp Tyr Ser Ala Met Ile His Pro Gly Arg Ile
        835                 840                 845

Val Ala Pro His Glu Tyr Gly Phe Gly Ile Glu Ala Asp Leu Ser Arg
    850                 855                 860

Glu Trp Val Arg Lys Ala Ser Ala Leu Gly Val Asp Tyr Tyr Asp Asn
865                 870                 875                 880

Val Arg Asn Val Glu Asn Val Ile Gly Thr Ser Met Lys Asp Val Leu
                885                 890                 895

Ile Gly Asp Ala Gln Ala Asn Thr Leu Met Gly Gln Gly Gly Asp Asp
            900                 905                 910

Thr Val Arg Gly Gly Asp Gly Asp Asp Leu Leu Phe Gly Gly Asp Gly
        915                 920                 925

Asn Asp Met Leu Tyr Gly Asp Ala Gly Asn Asp Thr Leu Tyr Gly Gly
    930                 935                 940

Leu Gly Asp Asp Thr Leu Glu Gly Gly Ala Gly Asn Asp Trp Phe Gly
945                 950                 955                 960

Gln Thr Gln Ala Arg Glu His Asp Val Leu Arg Gly Gly Asp Gly Val
                965                 970                 975

Asp Thr Val Asp Tyr Ser Gln Thr Gly Ala His Ala Gly Ile Ala Ala
            980                 985                 990

Gly Arg Ile Gly Leu Gly Ile Leu Ala Asp Leu Gly Ala Gly Arg Val
        995                 1000                1005

Asp Lys Leu Gly Glu Ala Gly Ser Ser Ala Tyr Asp Thr Val Ser
    1010                1015                1020

Gly Ile Glu Asn Val Val Gly Thr Glu Leu Ala Asp Arg Ile Thr
    1025                1030                1035

Gly Asp Ala Gln Ala Asn Val Leu Arg Gly Ala Gly Gly Ala Asp
    1040                1045                1050

Val Leu Ala Gly Gly Glu Gly Asp Asp Val Leu Leu Gly Gly Asp
    1055                1060                1065

Gly Asp Asp Gln Leu Ser Gly Asp Ala Gly Arg Asp Arg Leu Tyr
    1070                1075                1080

Gly Glu Ala Gly Asp Asp Trp Phe Phe Gln Asp Ala Ala Asn Ala
    1085                1090                1095

Gly Asn Leu Leu Asp Gly Gly Asp Gly Arg Asp Thr Val Asp Phe
    1100                1105                1110

Ser Gly Pro Gly Arg Gly Leu Asp Ala Gly Ala Lys Gly Val Phe
    1115                1120                1125

Leu Ser Leu Gly Lys Gly Phe Ala Ser Leu Met Asp Glu Pro Glu
    1130                1135                1140

Thr Ser Asn Val Leu Arg Asn Ile Glu Asn Ala Val Gly Ser Ala
    1145                1150                1155

Arg Asp Asp Val Leu Ile Gly Asp Ala Gly Ala Asn Val Leu Asn
    1160                1165                1170

Gly Leu Ala Gly Asn Asp Val Leu Ser Gly Gly Ala Gly Asp Asp
    1175                1180                1185

Val Leu Leu Gly Asp Glu Gly Ser Asp Leu Leu Ser Gly Asp Ala
    1190                1195                1200

Gly Asn Asp Asp Leu Phe Gly Gly Gln Gly Asp Asp Thr Tyr Leu
    1205                1210                1215

Phe Gly Val Gly Tyr Gly His Asp Thr Ile Tyr Glu Ser Gly Gly
    1220                1225                1230

Gly His Asp Thr Ile Arg Ile Asn Ala Gly Ala Asp Gln Leu Trp
    1235                1240                1245

Phe Ala Arg Gln Gly Asn Asp Leu Glu Ile Arg Ile Leu Gly Thr
    1250                1255                1260

Asp Asp Ala Leu Thr Val His Asp Trp Tyr Arg Asp Ala Asp His
    1265                1270                1275

Arg Val Glu Ile Ile His Ala Ala Asn Gln Ala Val Asp Gln Ala
    1280                1285                1290

Gly Ile Glu Lys Leu Val Glu Ala Met Ala Gln Tyr Pro Asp Pro
    1295                1300                1305

Gly Ala Ala Ala Ala Ala Pro Pro Ala Ala Arg Val Pro Asp Thr
    1310                1315                1320

Leu Met Gln Ser Leu Ala Val Asn Trp Arg
    1325                1330

<210> 3
<211> 1334
<212> PRT
<213> artificial

<220>
<223> CyaA fragment 373-1706, A680P V695P

<400> 3

Ser Lys Phe Ser Pro Asp Val Leu Glu Thr Val Pro Ala Ser Pro Gly
1               5                   10                  15

Leu Arg Arg Pro Ser Leu Gly Ala Val Glu Arg Gln Asp Ser Gly Tyr
            20                  25                  30

Asp Ser Leu Asp Gly Val Gly Ser Arg Ser Phe Ser Leu Gly Glu Val
        35                  40                  45

Ser Asp Met Ala Ala Val Glu Ala Ala Glu Leu Glu Met Thr Arg Gln
    50                  55                  60

Val Leu His Ala Gly Ala Arg Gln Asp Asp Ala Glu Pro Gly Val Ser
65                  70                  75                  80

Gly Ala Ser Ala His Trp Gly Gln Arg Ala Leu Gln Gly Ala Gln Ala
                85                  90                  95

Val Ala Ala Ala Gln Arg Leu Val His Ala Ile Ala Leu Met Thr Gln
            100                 105                 110

Phe Gly Arg Ala Gly Ser Thr Asn Thr Pro Gln Glu Ala Ala Ser Leu
        115                 120                 125

Ser Ala Ala Val Phe Gly Leu Gly Glu Ala Ser Ser Ala Val Ala Glu
    130                 135                 140

Thr Val Ser Gly Phe Phe Arg Gly Ser Ser Arg Trp Ala Gly Gly Phe
145                 150                 155                 160

Gly Val Ala Gly Gly Ala Met Ala Leu Gly Gly Gly Ile Ala Ala Ala
                165                 170                 175

Val Gly Ala Gly Met Ser Leu Thr Asp Asp Ala Pro Ala Gly Gln Lys
            180                 185                 190

Ala Ala Ala Gly Ala Glu Ile Ala Leu Gln Leu Thr Gly Gly Thr Val
        195                 200                 205

Glu Leu Ala Ser Ser Ile Ala Leu Ala Leu Ala Ala Ala Arg Gly Val
    210                 215                 220

Thr Ser Gly Leu Gln Val Ala Gly Ala Ser Ala Gly Ala Ala Ala Gly
225                 230                 235                 240

Ala Leu Ala Ala Ala Leu Ser Pro Met Glu Ile Tyr Gly Leu Val Gln
                245                 250                 255

Gln Ser His Tyr Ala Asp Gln Leu Asp Lys Leu Ala Gln Glu Ser Ser
            260                 265                 270

Ala Tyr Gly Tyr Glu Gly Asp Ala Leu Leu Ala Gln Leu Tyr Arg Asp
        275                 280                 285

Lys Thr Ala Ala Glu Gly Ala Val Ala Gly Val Ser Ala Val Leu Ser
    290                 295                 300

Thr Val Gly Pro Ala Val Ser Ile Ala Ala Ala Ala Ser Val Val Gly
305                 310                 315                 320

Ala Pro Pro Ala Val Val Thr Ser Leu Leu Thr Gly Ala Leu Asn Gly
                325                 330                 335

Ile Leu Arg Gly Val Gln Gln Pro Ile Ile Glu Lys Leu Ala Asn Asp
            340                 345                 350

Tyr Ala Arg Lys Ile Asp Glu Leu Gly Gly Pro Gln Ala Tyr Phe Glu
        355                 360                 365

Lys Asn Leu Gln Ala Arg His Glu Gln Leu Ala Asn Ser Asp Gly Leu
    370                 375                 380

Arg Lys Met Leu Ala Asp Leu Gln Ala Gly Trp Asn Ala Ser Ser Val
385                 390                 395                 400

Ile Gly Val Gln Thr Thr Glu Ile Ser Lys Ser Ala Leu Glu Leu Ala
                405                 410                 415

Ala Ile Thr Gly Asn Ala Asp Asn Leu Lys Ser Val Asp Val Phe Val
            420                 425                 430

Asp Arg Phe Val Gln Gly Glu Arg Val Ala Gly Gln Pro Val Val Leu
        435                 440                 445

Asp Val Ala Ala Gly Gly Ile Asp Ile Ala Ser Arg Lys Gly Glu Arg
    450                 455                 460

Pro Ala Leu Thr Phe Ile Thr Pro Leu Ala Ala Pro Gly Glu Glu Gln
465                 470                 475                 480

Arg Arg Arg Thr Lys Thr Gly Lys Ser Glu Phe Thr Thr Phe Val Glu
                485                 490                 495

Ile Val Gly Lys Gln Asp Arg Trp Arg Ile Arg Asp Gly Ala Ala Asp
            500                 505                 510

Thr Thr Ile Asp Leu Ala Lys Val Val Ser Gln Leu Val Asp Ala Asn
        515                 520                 525

Gly Val Leu Lys His Ser Ile Lys Leu Asp Val Ile Gly Gly Asp Gly
    530                 535                 540

Asp Asp Val Val Leu Ala Asn Ala Ser Arg Ile His Tyr Asp Gly Gly
545                 550                 555                 560

Ala Gly Thr Asn Thr Val Ser Tyr Ala Ala Leu Gly Arg Gln Asp Ser
                565                 570                 575

Ile Thr Val Ser Ala Asp Gly Glu Arg Phe Asn Val Arg Lys Gln Leu
            580                 585                 590

Asn Asn Ala Asn Val Tyr Arg Glu Gly Val Ala Thr Gln Thr Thr Ala
        595                 600                 605

Tyr Gly Lys Arg Thr Glu Asn Val Gln Tyr Arg His Val Glu Leu Ala
    610                 615                 620

Arg Val Gly Gln Leu Val Glu Val Asp Thr Leu Glu His Val Gln His
625                 630                 635                 640

Ile Ile Gly Gly Ala Gly Asn Asp Ser Ile Thr Gly Asn Ala His Asp
                645                 650                 655

Asn Phe Leu Ala Gly Gly Ser Gly Asp Asp Arg Leu Asp Gly Gly Ala
            660                 665                 670

Gly Asn Asp Thr Leu Val Gly Gly Glu Gly Gln Asn Thr Val Ile Gly
        675                 680                 685

Gly Ala Gly Asp Asp Val Phe Leu Gln Asp Leu Gly Val Trp Ser Asn
    690                 695                 700

Gln Leu Asp Gly Gly Ala Gly Val Asp Thr Val Lys Tyr Asn Val His
705                 710                 715                 720

Gln Pro Ser Glu Glu Arg Leu Glu Arg Met Gly Asp Thr Gly Ile His
                725                 730                 735

Ala Asp Leu Gln Lys Gly Thr Val Glu Lys Trp Pro Ala Leu Asn Leu
            740                 745                 750

Phe Ser Val Asp His Val Lys Asn Ile Glu Asn Leu His Gly Ser Arg

        755                 760                 765

Leu Asn Asp Arg Ile Ala Gly Asp Asp Gln Asp Asn Glu Leu Trp Gly
    770                 775                 780

His Asp Gly Asn Asp Thr Ile Arg Gly Arg Gly Gly Asp Asp Ile Leu
785                 790                 795                 800

Arg Gly Gly Leu Gly Leu Asp Thr Leu Tyr Gly Glu Asp Gly Asn Asp
                805                 810                 815

Ile Phe Leu Gln Asp Asp Glu Thr Val Ser Asp Asp Ile Asp Gly Gly
            820                 825                 830

Ala Gly Leu Asp Thr Val Asp Tyr Ser Ala Met Ile His Pro Gly Arg
        835                 840                 845

Ile Val Ala Pro His Glu Tyr Gly Phe Gly Ile Glu Ala Asp Leu Ser
    850                 855                 860

Arg Glu Trp Val Arg Lys Ala Ser Ala Leu Gly Val Asp Tyr Tyr Asp
865                 870                 875                 880

Asn Val Arg Asn Val Glu Asn Val Ile Gly Thr Ser Met Lys Asp Val
                885                 890                 895

Leu Ile Gly Asp Ala Gln Ala Asn Thr Leu Met Gly Gln Gly Gly Asp
            900                 905                 910

Asp Thr Val Arg Gly Gly Asp Gly Asp Asp Leu Leu Phe Gly Gly Asp
        915                 920                 925

Gly Asn Asp Met Leu Tyr Gly Asp Ala Gly Asn Asp Thr Leu Tyr Gly
    930                 935                 940

Gly Leu Gly Asp Asp Thr Leu Glu Gly Gly Ala Gly Asn Asp Trp Phe
945                 950                 955                 960

Gly Gln Thr Gln Ala Arg Glu His Asp Val Leu Arg Gly Gly Asp Gly
                965                 970                 975

Val Asp Thr Val Asp Tyr Ser Gln Thr Gly Ala His Ala Gly Ile Ala
            980                 985                 990

Ala Gly Arg Ile Gly Leu Gly Ile Leu Ala Asp Leu Gly Ala Gly Arg
        995                 1000                1005

Val Asp Lys Leu Gly Glu Ala Gly Ser Ser Ala Tyr Asp Thr Val
    1010                1015                1020

Ser Gly Ile Glu Asn Val Val Gly Thr Glu Leu Ala Asp Arg Ile
    1025                1030                1035

Thr Gly Asp Ala Gln Ala Asn Val Leu Arg Gly Ala Gly Gly Ala
    1040                1045                1050

Asp Val Leu Ala Gly Gly Glu Gly Asp Asp Val Leu Leu Gly Gly
    1055                1060                1065

Asp Gly Asp Asp Gln Leu Ser Gly Asp Ala Gly Arg Asp Arg Leu
    1070                1075                1080

Tyr Gly Glu Ala Gly Asp Asp Trp Phe Phe Gln Asp Ala Ala Asn
    1085                1090                1095

Ala Gly Asn Leu Leu Asp Gly Gly Asp Gly Arg Asp Thr Val Asp
    1100                1105                1110

Phe Ser Gly Pro Gly Arg Gly Leu Asp Ala Gly Ala Lys Gly Val
    1115                1120                1125

Phe Leu Ser Leu Gly Lys Gly Phe Ala Ser Leu Met Asp Glu Pro
    1130                1135                1140

Glu Thr Ser Asn Val Leu Arg Asn Ile Glu Asn Ala Val Gly Ser
    1145                1150                1155

Ala Arg Asp Asp Val Leu Ile Gly Asp Ala Gly Ala Asn Val Leu
    1160                1165                1170

Asn Gly Leu Ala Gly Asn Asp Val Leu Ser Gly Gly Ala Gly Asp
    1175                1180                1185

Asp Val Leu Leu Gly Asp Glu Gly Ser Asp Leu Leu Ser Gly Asp
    1190                1195                1200

Ala Gly Asn Asp Asp Leu Phe Gly Gly Gln Gly Asp Asp Thr Tyr
    1205                1210                1215

Leu Phe Gly Val Gly Tyr Gly His Asp Thr Ile Tyr Glu Ser Gly
    1220                1225                1230

Gly Gly His Asp Thr Ile Arg Ile Asn Ala Gly Ala Asp Gln Leu
    1235                1240                1245

Trp Phe Ala Arg Gln Gly Asn Asp Leu Glu Ile Arg Ile Leu Gly
    1250                1255                1260

Thr Asp Asp Ala Leu Thr Val His Asp Trp Tyr Arg Asp Ala Asp
    1265                1270                1275

His Arg Val Glu Ile Ile His Ala Ala Asn Gln Ala Val Asp Gln
    1280                1285                1290

Ala Gly Ile Glu Lys Leu Val Glu Ala Met Ala Gln Tyr Pro Asp
    1295                1300                1305

Pro Gly Ala Ala Ala Ala Ala Pro Pro Ala Ala Arg Val Pro Asp
    1310                1315                1320

Thr Leu Met Gln Ser Leu Ala Val Asn Trp Arg
    1325                1330

<210> 4
<211> 1335
<212> PRT
<213> artificial

<220>
<223> CyaA fragment 372-1706, A680P V695P

<400> 4

Arg Ser Lys Phe Ser Pro Asp Val Leu Glu Thr Val Pro Ala Ser Pro
1               5                   10                  15

Gly Leu Arg Arg Pro Ser Leu Gly Ala Val Glu Arg Gln Asp Ser Gly
            20                  25                  30

Tyr Asp Ser Leu Asp Gly Val Gly Ser Arg Ser Phe Ser Leu Gly Glu
        35                  40                  45

Val Ser Asp Met Ala Ala Val Glu Ala Ala Glu Leu Glu Met Thr Arg
    50                  55                  60

Gln Val Leu His Ala Gly Ala Arg Gln Asp Asp Ala Glu Pro Gly Val
65                  70                  75                  80

Ser Gly Ala Ser Ala His Trp Gly Gln Arg Ala Leu Gln Gly Ala Gln
                85                  90                  95

Ala Val Ala Ala Ala Gln Arg Leu Val His Ala Ile Ala Leu Met Thr
            100                 105                 110

Gln Phe Gly Arg Ala Gly Ser Thr Asn Thr Pro Gln Glu Ala Ala Ser
        115                 120                 125

Leu Ser Ala Ala Val Phe Gly Leu Gly Glu Ala Ser Ser Ala Val Ala
    130                 135                 140

Glu Thr Val Ser Gly Phe Phe Arg Gly Ser Ser Arg Trp Ala Gly Gly
145                 150                 155                 160

Phe Gly Val Ala Gly Gly Ala Met Ala Leu Gly Gly Gly Ile Ala Ala
                165                 170                 175

Ala Val Gly Ala Gly Met Ser Leu Thr Asp Asp Ala Pro Ala Gly Gln
            180                 185                 190

Lys Ala Ala Ala Gly Ala Glu Ile Ala Leu Gln Leu Thr Gly Gly Thr
        195                 200                 205

Val Glu Leu Ala Ser Ser Ile Ala Leu Ala Leu Ala Ala Ala Arg Gly
    210                 215                 220

Val Thr Ser Gly Leu Gln Val Ala Gly Ala Ser Ala Gly Ala Ala Ala
225                 230                 235                 240

Gly Ala Leu Ala Ala Ala Leu Ser Pro Met Glu Ile Tyr Gly Leu Val
                245                 250                 255

Gln Gln Ser His Tyr Ala Asp Gln Leu Asp Lys Leu Ala Gln Glu Ser
            260                 265                 270

Ser Ala Tyr Gly Tyr Glu Gly Asp Ala Leu Leu Ala Gln Leu Tyr Arg
        275                 280                 285

Asp Lys Thr Ala Ala Glu Gly Ala Val Ala Gly Val Ser Ala Val Leu
    290                 295                 300

Ser Thr Val Gly Pro Ala Val Ser Ile Ala Ala Ala Ala Ser Val Val
305                 310                 315                 320

Gly Ala Pro Pro Ala Val Val Thr Ser Leu Leu Thr Gly Ala Leu Asn
                325                 330                 335

Gly Ile Leu Arg Gly Val Gln Gln Pro Ile Ile Glu Lys Leu Ala Asn
            340                 345                 350

Asp Tyr Ala Arg Lys Ile Asp Glu Leu Gly Gly Pro Gln Ala Tyr Phe
        355                 360                 365

Glu Lys Asn Leu Gln Ala Arg His Glu Gln Leu Ala Asn Ser Asp Gly
    370                 375                 380

Leu Arg Lys Met Leu Ala Asp Leu Gln Ala Gly Trp Asn Ala Ser Ser
385                 390                 395                 400

Val Ile Gly Val Gln Thr Thr Glu Ile Ser Lys Ser Ala Leu Glu Leu
                405                 410                 415

Ala Ala Ile Thr Gly Asn Ala Asp Asn Leu Lys Ser Val Asp Val Phe
            420                 425                 430

Val Asp Arg Phe Val Gln Gly Glu Arg Val Ala Gly Gln Pro Val Val
        435                 440                 445

Leu Asp Val Ala Ala Gly Gly Ile Asp Ile Ala Ser Arg Lys Gly Glu
    450                 455                 460

Arg Pro Ala Leu Thr Phe Ile Thr Pro Leu Ala Ala Pro Gly Glu Glu
465                 470                 475                 480

Gln Arg Arg Arg Thr Lys Thr Gly Lys Ser Glu Phe Thr Thr Phe Val
                485                 490                 495

Glu Ile Val Gly Lys Gln Asp Arg Trp Arg Ile Arg Asp Gly Ala Ala
            500                 505                 510

Asp Thr Thr Ile Asp Leu Ala Lys Val Val Ser Gln Leu Val Asp Ala
        515                 520                 525

Asn Gly Val Leu Lys His Ser Ile Lys Leu Asp Val Ile Gly Gly Asp
    530                 535                 540

Gly Asp Asp Val Val Leu Ala Asn Ala Ser Arg Ile His Tyr Asp Gly
545                 550                 555                 560

Gly Ala Gly Thr Asn Thr Val Ser Tyr Ala Ala Leu Gly Arg Gln Asp
                565                 570                 575

Ser Ile Thr Val Ser Ala Asp Gly Glu Arg Phe Asn Val Arg Lys Gln
            580                 585                 590

Leu Asn Asn Ala Asn Val Tyr Arg Glu Gly Val Ala Thr Gln Thr Thr
        595                 600                 605

Ala Tyr Gly Lys Arg Thr Glu Asn Val Gln Tyr Arg His Val Glu Leu
    610                 615                 620

Ala Arg Val Gly Gln Leu Val Glu Val Asp Thr Leu Glu His Val Gln
625                 630                 635                 640

His Ile Ile Gly Gly Ala Gly Asn Asp Ser Ile Thr Gly Asn Ala His
                645                 650                 655

Asp Asn Phe Leu Ala Gly Gly Ser Gly Asp Asp Arg Leu Asp Gly Gly
            660                 665                 670

Ala Gly Asn Asp Thr Leu Val Gly Gly Glu Gly Gln Asn Thr Val Ile
        675                 680                 685

Gly Gly Ala Gly Asp Asp Val Phe Leu Gln Asp Leu Gly Val Trp Ser
    690                 695                 700

Asn Gln Leu Asp Gly Gly Ala Gly Val Asp Thr Val Lys Tyr Asn Val
705                 710                 715                 720

His Gln Pro Ser Glu Glu Arg Leu Glu Arg Met Gly Asp Thr Gly Ile
                725                 730                 735

His Ala Asp Leu Gln Lys Gly Thr Val Glu Lys Trp Pro Ala Leu Asn
            740                 745                 750

Leu Phe Ser Val Asp His Val Lys Asn Ile Glu Asn Leu His Gly Ser
        755                 760                 765

Arg Leu Asn Asp Arg Ile Ala Gly Asp Asp Gln Asp Asn Glu Leu Trp
    770                 775                 780

Gly His Asp Gly Asn Asp Thr Ile Arg Gly Arg Gly Gly Asp Asp Ile
785                 790                 795                 800

Leu Arg Gly Gly Leu Gly Leu Asp Thr Leu Tyr Gly Glu Asp Gly Asn
                805                 810                 815

Asp Ile Phe Leu Gln Asp Asp Glu Thr Val Ser Asp Asp Ile Asp Gly
            820                 825                 830

Gly Ala Gly Leu Asp Thr Val Asp Tyr Ser Ala Met Ile His Pro Gly
        835                 840                 845

Arg Ile Val Ala Pro His Glu Tyr Gly Phe Gly Ile Glu Ala Asp Leu
    850                 855                 860

Ser Arg Glu Trp Val Arg Lys Ala Ser Ala Leu Gly Val Asp Tyr Tyr

865                 870                 875                 880

Asp Asn Val Arg Asn Val Glu Asn Val Ile Gly Thr Ser Met Lys Asp
                885                 890                 895

Val Leu Ile Gly Asp Ala Gln Ala Asn Thr Leu Met Gly Gln Gly Gly
            900                 905                 910

Asp Asp Thr Val Arg Gly Gly Asp Gly Asp Asp Leu Leu Phe Gly Gly
        915                 920                 925

Asp Gly Asn Asp Met Leu Tyr Gly Asp Ala Gly Asn Asp Thr Leu Tyr
    930                 935                 940

Gly Gly Leu Gly Asp Asp Thr Leu Glu Gly Gly Ala Gly Asn Asp Trp
945                 950                 955                 960

Phe Gly Gln Thr Gln Ala Arg Glu His Asp Val Leu Arg Gly Gly Asp
                965                 970                 975

Gly Val Asp Thr Val Asp Tyr Ser Gln Thr Gly Ala His Ala Gly Ile
            980                 985                 990

Ala Ala Gly Arg Ile Gly Leu Gly  Ile Leu Ala Asp Leu  Gly Ala Gly
        995                 1000                 1005

Arg Val  Asp Lys Leu Gly Glu  Ala Gly Ser Ser Ala  Tyr Asp Thr
    1010                 1015                 1020

Val Ser  Gly Ile Glu Asn Val  Val Gly Thr Glu Leu  Ala Asp Arg
    1025                 1030                 1035

Ile Thr  Gly Asp Ala Gln Ala  Asn Val Leu Arg Gly  Ala Gly Gly
    1040                 1045                 1050

Ala Asp  Val Leu Ala Gly Gly  Glu Gly Asp Asp Val  Leu Leu Gly
    1055                 1060                 1065

Gly Asp  Gly Asp Asp Gln Leu  Ser Gly Asp Ala Gly  Arg Asp Arg
    1070                 1075                 1080

Leu Tyr  Gly Glu Ala Gly Asp  Asp Trp Phe Phe Gln  Asp Ala Ala
    1085                 1090                 1095

Asn Ala  Gly Asn Leu Leu Asp  Gly Gly Asp Gly Arg  Asp Thr Val
    1100                 1105                 1110

Asp Phe Ser Gly Pro Gly Arg Gly Leu Asp Ala Gly Ala Lys Gly
    1115                1120                1125

Val Phe Leu Ser Leu Gly Lys Gly Phe Ala Ser Leu Met Asp Glu
    1130                1135                1140

Pro Glu Thr Ser Asn Val Leu Arg Asn Ile Glu Asn Ala Val Gly
    1145                1150                1155

Ser Ala Arg Asp Asp Val Leu Ile Gly Asp Ala Gly Ala Asn Val
    1160                1165                1170

Leu Asn Gly Leu Ala Gly Asn Asp Val Leu Ser Gly Gly Ala Gly
    1175                1180                1185

Asp Asp Val Leu Leu Gly Asp Glu Gly Ser Asp Leu Leu Ser Gly
    1190                1195                1200

Asp Ala Gly Asn Asp Asp Leu Phe Gly Gly Gln Gly Asp Asp Thr
    1205                1210                1215

Tyr Leu Phe Gly Val Gly Tyr Gly His Asp Thr Ile Tyr Glu Ser
    1220                1225                1230

Gly Gly Gly His Asp Thr Ile Arg Ile Asn Ala Gly Ala Asp Gln
    1235                1240                1245

Leu Trp Phe Ala Arg Gln Gly Asn Asp Leu Glu Ile Arg Ile Leu
    1250                1255                1260

Gly Thr Asp Asp Ala Leu Thr Val His Asp Trp Tyr Arg Asp Ala
    1265                1270                1275

Asp His Arg Val Glu Ile Ile His Ala Ala Asn Gln Ala Val Asp
    1280                1285                1290

Gln Ala Gly Ile Glu Lys Leu Val Glu Ala Met Ala Gln Tyr Pro
    1295                1300                1305

Asp Pro Gly Ala Ala Ala Ala Ala Pro Pro Ala Ala Arg Val Pro
    1310                1315                1320

Asp Thr Leu Met Gln Ser Leu Ala Val Asn Trp Arg
    1325                1330                1335

<210>  5
<211>  1706
<212>  PRT

<213>  artificial

<220>
<223>  CyaA mutated A680P V695P

<400>  5

Met Gln Gln Ser His Gln Ala Gly Tyr Ala Asn Ala Ala Asp Arg Glu
1               5                   10                  15

Ser Gly Ile Pro Ala Ala Val Leu Asp Gly Ile Lys Ala Val Ala Lys
            20                  25                  30

Glu Lys Asn Ala Thr Leu Met Phe Arg Leu Val Asn Pro His Ser Thr
        35                  40                  45

Ser Leu Ile Ala Glu Gly Val Ala Thr Lys Gly Leu Gly Val His Ala
    50                  55                  60

Lys Ser Ser Asp Trp Gly Leu Gln Ala Gly Tyr Ile Pro Val Asn Pro
65                  70                  75                  80

Asn Leu Ser Lys Leu Phe Gly Arg Ala Pro Glu Val Ile Ala Arg Ala
                85                  90                  95

Asp Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu
        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala
145                 150                 155                 160

Val Gln Tyr Arg Arg Lys Gly Gly Asp Asp Phe Glu Ala Val Lys Val
                165                 170                 175

Ile Gly Asn Ala Ala Gly Ile Pro Leu Thr Ala Asp Ile Asp Met Phe
            180                 185                 190

Ala Ile Met Pro His Leu Ser Asn Phe Arg Asp Ser Ala Arg Ser Ser
        195                 200                 205

Val Thr Ser Gly Asp Ser Val Thr Asp Tyr Leu Ala Arg Thr Arg Arg
    210                 215                 220

Ala Ala Ser Glu Ala Thr Gly Gly Leu Asp Arg Glu Arg Ile Asp Leu
225                 230                 235                 240

Leu Trp Lys Ile Ala Arg Ala Gly Ala Arg Ser Ala Val Gly Thr Glu
                245                 250                 255

Ala Arg Arg Gln Phe Arg Tyr Asp Gly Asp Met Asn Ile Gly Val Ile
            260                 265                 270

Thr Asp Phe Glu Leu Glu Val Arg Asn Ala Leu Asn Arg Arg Ala His
        275                 280                 285

Ala Val Gly Ala Gln Asp Val Val Gln His Gly Thr Glu Gln Asn Asn
    290                 295                 300

Pro Phe Pro Glu Ala Asp Glu Lys Ile Phe Val Val Ser Ala Thr Gly
305                 310                 315                 320

Glu Ser Gln Met Leu Thr Arg Gly Gln Leu Lys Glu Tyr Ile Gly Gln
                325                 330                 335

Gln Arg Gly Glu Gly Tyr Val Phe Tyr Glu Asn Arg Ala Tyr Gly Val
            340                 345                 350

Ala Gly Lys Ser Leu Phe Asp Asp Gly Leu Gly Ala Ala Pro Gly Val
        355                 360                 365

Pro Ser Gly Arg Ser Lys Phe Ser Pro Asp Val Leu Glu Thr Val Pro
    370                 375                 380

Ala Ser Pro Gly Leu Arg Arg Pro Ser Leu Gly Ala Val Glu Arg Gln
385                 390                 395                 400

Asp Ser Gly Tyr Asp Ser Leu Asp Gly Val Gly Ser Arg Ser Phe Ser
                405                 410                 415

Leu Gly Glu Val Ser Asp Met Ala Ala Val Glu Ala Ala Glu Leu Glu
            420                 425                 430

Met Thr Arg Gln Val Leu His Ala Gly Ala Arg Gln Asp Asp Ala Glu
        435                 440                 445

Pro Gly Val Ser Gly Ala Ser Ala His Trp Gly Gln Arg Ala Leu Gln
    450                 455                 460

Gly Ala Gln Ala Val Ala Ala Ala Gln Arg Leu Val His Ala Ile Ala
465                 470                 475                 480

Leu Met Thr Gln Phe Gly Arg Ala Gly Ser Thr Asn Thr Pro Gln Glu
                485                 490                 495

Ala Ala Ser Leu Ser Ala Ala Val Phe Gly Leu Gly Glu Ala Ser Ser
            500                 505                 510

Ala Val Ala Glu Thr Val Ser Gly Phe Phe Arg Gly Ser Ser Arg Trp
        515                 520                 525

Ala Gly Gly Phe Gly Val Ala Gly Gly Ala Met Ala Leu Gly Gly Gly
    530                 535                 540

Ile Ala Ala Ala Val Gly Ala Gly Met Ser Leu Thr Asp Asp Ala Pro
545                 550                 555                 560

Ala Gly Gln Lys Ala Ala Ala Gly Ala Glu Ile Ala Leu Gln Leu Thr
                565                 570                 575

Gly Gly Thr Val Glu Leu Ala Ser Ser Ile Ala Leu Ala Leu Ala Ala
            580                 585                 590

Ala Arg Gly Val Thr Ser Gly Leu Gln Val Ala Gly Ala Ser Ala Gly
        595                 600                 605

Ala Ala Ala Gly Ala Leu Ala Ala Ala Leu Ser Pro Met Glu Ile Tyr
    610                 615                 620

Gly Leu Val Gln Gln Ser His Tyr Ala Asp Gln Leu Asp Lys Leu Ala
625                 630                 635                 640

Gln Glu Ser Ser Ala Tyr Gly Tyr Glu Gly Asp Ala Leu Leu Ala Gln
                645                 650                 655

Leu Tyr Arg Asp Lys Thr Ala Ala Glu Gly Ala Val Ala Gly Val Ser
            660                 665                 670

Ala Val Leu Ser Thr Val Gly Pro Ala Val Ser Ile Ala Ala Ala Ala
        675                 680                 685

Ser Val Val Gly Ala Pro Pro Ala Val Val Thr Ser Leu Leu Thr Gly
    690                 695                 700

Ala Leu Asn Gly Ile Leu Arg Gly Val Gln Gln Pro Ile Ile Glu Lys
705                 710                 715                 720

Leu Ala Asn Asp Tyr Ala Arg Lys Ile Asp Glu Leu Gly Gly Pro Gln
                725                 730                 735

Ala Tyr Phe Glu Lys Asn Leu Gln Ala Arg His Glu Gln Leu Ala Asn
        740                 745                 750

Ser Asp Gly Leu Arg Lys Met Leu Ala Asp Leu Gln Ala Gly Trp Asn
        755                 760                 765

Ala Ser Ser Val Ile Gly Val Gln Thr Thr Glu Ile Ser Lys Ser Ala
    770                 775                 780

Leu Glu Leu Ala Ala Ile Thr Gly Asn Ala Asp Asn Leu Lys Ser Val
785                 790                 795                 800

Asp Val Phe Val Asp Arg Phe Val Gln Gly Glu Arg Val Ala Gly Gln
                805                 810                 815

Pro Val Val Leu Asp Val Ala Ala Gly Gly Ile Asp Ile Ala Ser Arg
            820                 825                 830

Lys Gly Glu Arg Pro Ala Leu Thr Phe Ile Thr Pro Leu Ala Ala Pro
        835                 840                 845

Gly Glu Glu Gln Arg Arg Arg Thr Lys Thr Gly Lys Ser Glu Phe Thr
    850                 855                 860

Thr Phe Val Glu Ile Val Gly Lys Gln Asp Arg Trp Arg Ile Arg Asp
865                 870                 875                 880

Gly Ala Ala Asp Thr Thr Ile Asp Leu Ala Lys Val Val Ser Gln Leu
                885                 890                 895

Val Asp Ala Asn Gly Val Leu Lys His Ser Ile Lys Leu Asp Val Ile
            900                 905                 910

Gly Gly Asp Gly Asp Asp Val Val Leu Ala Asn Ala Ser Arg Ile His
        915                 920                 925

Tyr Asp Gly Gly Ala Gly Thr Asn Thr Val Ser Tyr Ala Ala Leu Gly
    930                 935                 940

Arg Gln Asp Ser Ile Thr Val Ser Ala Asp Gly Glu Arg Phe Asn Val
945                 950                 955                 960

Arg Lys Gln Leu Asn Asn Ala Asn Val Tyr Arg Glu Gly Val Ala Thr
                965                 970                 975

Gln Thr Thr Ala Tyr Gly Lys Arg Thr Glu Asn Val Gln Tyr Arg His

            980                 985                 990

Val Glu Leu Ala Arg Val Gly Gln Leu Val Glu Val Asp Thr Leu Glu
        995                 1000                1005

His Val Gln His Ile Ile Gly Gly Ala Gly Asn Asp Ser Ile Thr
    1010                1015                1020

Gly Asn Ala His Asp Asn Phe Leu Ala Gly Gly Ser Gly Asp Asp
    1025                1030                1035

Arg Leu Asp Gly Gly Ala Gly Asn Asp Thr Leu Val Gly Gly Glu
    1040                1045                1050

Gly Gln Asn Thr Val Ile Gly Gly Ala Gly Asp Asp Val Phe Leu
    1055                1060                1065

Gln Asp Leu Gly Val Trp Ser Asn Gln Leu Asp Gly Gly Ala Gly
    1070                1075                1080

Val Asp Thr Val Lys Tyr Asn Val His Gln Pro Ser Glu Glu Arg
    1085                1090                1095

Leu Glu Arg Met Gly Asp Thr Gly Ile His Ala Asp Leu Gln Lys
    1100                1105                1110

Gly Thr Val Glu Lys Trp Pro Ala Leu Asn Leu Phe Ser Val Asp
    1115                1120                1125

His Val Lys Asn Ile Glu Asn Leu His Gly Ser Arg Leu Asn Asp
    1130                1135                1140

Arg Ile Ala Gly Asp Asp Gln Asp Asn Glu Leu Trp Gly His Asp
    1145                1150                1155

Gly Asn Asp Thr Ile Arg Gly Arg Gly Gly Asp Asp Ile Leu Arg
    1160                1165                1170

Gly Gly Leu Gly Leu Asp Thr Leu Tyr Gly Glu Asp Gly Asn Asp
    1175                1180                1185

Ile Phe Leu Gln Asp Asp Glu Thr Val Ser Asp Asp Ile Asp Gly
    1190                1195                1200

Gly Ala Gly Leu Asp Thr Val Asp Tyr Ser Ala Met Ile His Pro
    1205                1210                1215

Gly Arg Ile Val Ala Pro His Glu Tyr Gly Phe Gly Ile Glu Ala
    1220                1225                1230

Asp Leu Ser Arg Glu Trp Val Arg Lys Ala Ser Ala Leu Gly Val
    1235                1240                1245

Asp Tyr Tyr Asp Asn Val Arg Asn Val Glu Asn Val Ile Gly Thr
    1250                1255                1260

Ser Met Lys Asp Val Leu Ile Gly Asp Ala Gln Ala Asn Thr Leu
    1265                1270                1275

Met Gly Gln Gly Gly Asp Asp Thr Val Arg Gly Gly Asp Gly Asp
    1280                1285                1290

Asp Leu Leu Phe Gly Gly Asp Gly Asn Asp Met Leu Tyr Gly Asp
    1295                1300                1305

Ala Gly Asn Asp Thr Leu Tyr Gly Gly Leu Gly Asp Asp Thr Leu
    1310                1315                1320

Glu Gly Gly Ala Gly Asn Asp Trp Phe Gly Gln Thr Gln Ala Arg
    1325                1330                1335

Glu His Asp Val Leu Arg Gly Gly Asp Gly Val Asp Thr Val Asp
    1340                1345                1350

Tyr Ser Gln Thr Gly Ala His Ala Gly Ile Ala Ala Gly Arg Ile
    1355                1360                1365

Gly Leu Gly Ile Leu Ala Asp Leu Gly Ala Gly Arg Val Asp Lys
    1370                1375                1380

Leu Gly Glu Ala Gly Ser Ser Ala Tyr Asp Thr Val Ser Gly Ile
    1385                1390                1395

Glu Asn Val Val Gly Thr Glu Leu Ala Asp Arg Ile Thr Gly Asp
    1400                1405                1410

Ala Gln Ala Asn Val Leu Arg Gly Ala Gly Gly Ala Asp Val Leu
    1415                1420                1425

Ala Gly Gly Glu Gly Asp Asp Val Leu Leu Gly Gly Asp Gly Asp
    1430                1435                1440

Asp Gln Leu Ser Gly Asp Ala Gly Arg Asp Arg Leu Tyr Gly Glu
    1445                1450                1455

Ala Gly Asp Asp Trp Phe Phe Gln Asp Ala Ala Asn Ala Gly Asn
    1460                1465                1470

Leu Leu Asp Gly Gly Asp Gly Arg Asp Thr Val Asp Phe Ser Gly
    1475                1480                1485

Pro Gly Arg Gly Leu Asp Ala Gly Ala Lys Gly Val Phe Leu Ser
    1490                1495                1500

Leu Gly Lys Gly Phe Ala Ser Leu Met Asp Glu Pro Glu Thr Ser
    1505                1510                1515

Asn Val Leu Arg Asn Ile Glu Asn Ala Val Gly Ser Ala Arg Asp
    1520                1525                1530

Asp Val Leu Ile Gly Asp Ala Gly Ala Asn Val Leu Asn Gly Leu
    1535                1540                1545

Ala Gly Asn Asp Val Leu Ser Gly Gly Ala Gly Asp Asp Val Leu
    1550                1555                1560

Leu Gly Asp Glu Gly Ser Asp Leu Leu Ser Gly Asp Ala Gly Asn
    1565                1570                1575

Asp Asp Leu Phe Gly Gly Gln Gly Asp Asp Thr Tyr Leu Phe Gly
    1580                1585                1590

Val Gly Tyr Gly His Asp Thr Ile Tyr Glu Ser Gly Gly Gly His
    1595                1600                1605

Asp Thr Ile Arg Ile Asn Ala Gly Ala Asp Gln Leu Trp Phe Ala
    1610                1615                1620

Arg Gln Gly Asn Asp Leu Glu Ile Arg Ile Leu Gly Thr Asp Asp
    1625                1630                1635

Ala Leu Thr Val His Asp Trp Tyr Arg Asp Ala Asp His Arg Val
    1640                1645                1650

Glu Ile Ile His Ala Ala Asn Gln Ala Val Asp Gln Ala Gly Ile
    1655                1660                1665

Glu Lys Leu Val Glu Ala Met Ala Gln Tyr Pro Asp Pro Gly Ala
    1670                1675                1680

Ala Ala Ala Ala Pro Pro Ala Ala Arg Val Pro Asp Thr Leu Met
    1685                1690                1695

Gln Ser Leu Ala Val Asn Trp Arg
    1700                1705

<210> 6
<211> 1708
<212> PRT
<213> artificial

<220>
<223> CyaA mutated A680P V695P, GS insert between 188-189 of CyaA

<400> 6

Met Gln Gln Ser His Gln Ala Gly Tyr Ala Asn Ala Ala Asp Arg Glu
1               5                   10                  15

Ser Gly Ile Pro Ala Ala Val Leu Asp Gly Ile Lys Ala Val Ala Lys
            20                  25                  30

Glu Lys Asn Ala Thr Leu Met Phe Arg Leu Val Asn Pro His Ser Thr
        35                  40                  45

Ser Leu Ile Ala Glu Gly Val Ala Thr Lys Gly Leu Gly Val His Ala
    50                  55                  60

Lys Ser Ser Asp Trp Gly Leu Gln Ala Gly Tyr Ile Pro Val Asn Pro
65                  70                  75                  80

Asn Leu Ser Lys Leu Phe Gly Arg Ala Pro Glu Val Ile Ala Arg Ala
                85                  90                  95

Asp Asn Asp Val Asn Ser Ser Leu Ala His Gly His Thr Ala Val Asp
            100                 105                 110

Leu Thr Leu Ser Lys Glu Arg Leu Asp Tyr Leu Arg Gln Ala Gly Leu
        115                 120                 125

Val Thr Gly Met Ala Asp Gly Val Val Ala Ser Asn His Ala Gly Tyr
    130                 135                 140

Glu Gln Phe Glu Phe Arg Val Lys Glu Thr Ser Asp Gly Arg Tyr Ala
145                 150                 155                 160

Val Gln Tyr Arg Arg Lys Gly Gly Asp Asp Phe Glu Ala Val Lys Val
                165                 170                 175

Ile Gly Asn Ala Ala Gly Ile Pro Leu Thr Ala Asp Gly Ser Ile Asp
            180                 185                 190

Met Phe Ala Ile Met Pro His Leu Ser Asn Phe Arg Asp Ser Ala Arg
        195                 200                 205

Ser Ser Val Thr Ser Gly Asp Ser Val Thr Asp Tyr Leu Ala Arg Thr
    210                 215                 220

Arg Arg Ala Ala Ser Glu Ala Thr Gly Gly Leu Asp Arg Glu Arg Ile
225                 230                 235                 240

Asp Leu Leu Trp Lys Ile Ala Arg Ala Gly Ala Arg Ser Ala Val Gly
                245                 250                 255

Thr Glu Ala Arg Arg Gln Phe Arg Tyr Asp Gly Asp Met Asn Ile Gly
            260                 265                 270

Val Ile Thr Asp Phe Glu Leu Glu Val Arg Asn Ala Leu Asn Arg Arg
        275                 280                 285

Ala His Ala Val Gly Ala Gln Asp Val Val Gln His Gly Thr Glu Gln
    290                 295                 300

Asn Asn Pro Phe Pro Glu Ala Asp Glu Lys Ile Phe Val Val Ser Ala
305                 310                 315                 320

Thr Gly Glu Ser Gln Met Leu Thr Arg Gly Gln Leu Lys Glu Tyr Ile
                325                 330                 335

Gly Gln Gln Arg Gly Glu Gly Tyr Val Phe Tyr Glu Asn Arg Ala Tyr
            340                 345                 350

Gly Val Ala Gly Lys Ser Leu Phe Asp Asp Gly Leu Gly Ala Ala Pro
        355                 360                 365

Gly Val Pro Ser Gly Arg Ser Lys Phe Ser Pro Asp Val Leu Glu Thr
    370                 375                 380

Val Pro Ala Ser Pro Gly Leu Arg Arg Pro Ser Leu Gly Ala Val Glu
385                 390                 395                 400

Arg Gln Asp Ser Gly Tyr Asp Ser Leu Asp Gly Val Gly Ser Arg Ser
                405                 410                 415

Phe Ser Leu Gly Glu Val Ser Asp Met Ala Ala Val Glu Ala Ala Glu
            420                 425                 430

Leu Glu Met Thr Arg Gln Val Leu His Ala Gly Ala Arg Gln Asp Asp
        435                 440                 445

Ala Glu Pro Gly Val Ser Gly Ala Ser Ala His Trp Gly Gln Arg Ala
    450                 455                 460

Leu Gln Gly Ala Gln Ala Val Ala Ala Ala Gln Arg Leu Val His Ala
465                 470                 475                 480

Ile Ala Leu Met Thr Gln Phe Gly Arg Ala Gly Ser Thr Asn Thr Pro
                485                 490                 495

Gln Glu Ala Ala Ser Leu Ser Ala Ala Val Phe Gly Leu Gly Glu Ala
            500                 505                 510

Ser Ser Ala Val Ala Glu Thr Val Ser Gly Phe Phe Arg Gly Ser Ser
        515                 520                 525

Arg Trp Ala Gly Gly Phe Gly Val Ala Gly Gly Ala Met Ala Leu Gly
    530                 535                 540

Gly Gly Ile Ala Ala Ala Val Gly Ala Gly Met Ser Leu Thr Asp Asp
545                 550                 555                 560

Ala Pro Ala Gly Gln Lys Ala Ala Ala Gly Ala Glu Ile Ala Leu Gln
                565                 570                 575

Leu Thr Gly Gly Thr Val Glu Leu Ala Ser Ser Ile Ala Leu Ala Leu
            580                 585                 590

Ala Ala Ala Arg Gly Val Thr Ser Gly Leu Gln Val Ala Gly Ala Ser
        595                 600                 605

Ala Gly Ala Ala Ala Gly Ala Leu Ala Ala Ala Leu Ser Pro Met Glu
    610                 615                 620

Ile Tyr Gly Leu Val Gln Gln Ser His Tyr Ala Asp Gln Leu Asp Lys
625                 630                 635                 640

Leu Ala Gln Glu Ser Ser Ala Tyr Gly Tyr Glu Gly Asp Ala Leu Leu
                645                 650                 655

Ala Gln Leu Tyr Arg Asp Lys Thr Ala Ala Glu Gly Ala Val Ala Gly
            660                 665                 670

Val Ser Ala Val Leu Ser Thr Val Gly Pro Ala Val Ser Ile Ala Ala
        675                 680                 685

Ala Ala Ser Val Val Gly Ala Pro Pro Ala Val Val Thr Ser Leu Leu

690                 695                 700

Thr Gly Ala Leu Asn Gly Ile Leu Arg Gly Val Gln Gln Pro Ile Ile
705                 710                 715                 720

Glu Lys Leu Ala Asn Asp Tyr Ala Arg Lys Ile Asp Glu Leu Gly Gly
                725                 730                 735

Pro Gln Ala Tyr Phe Glu Lys Asn Leu Gln Ala Arg His Glu Gln Leu
            740                 745                 750

Ala Asn Ser Asp Gly Leu Arg Lys Met Leu Ala Asp Leu Gln Ala Gly
        755                 760                 765

Trp Asn Ala Ser Ser Val Ile Gly Val Gln Thr Thr Glu Ile Ser Lys
    770                 775                 780

Ser Ala Leu Glu Leu Ala Ala Ile Thr Gly Asn Ala Asp Asn Leu Lys
785                 790                 795                 800

Ser Val Asp Val Phe Val Asp Arg Phe Val Gln Gly Glu Arg Val Ala
                805                 810                 815

Gly Gln Pro Val Val Leu Asp Val Ala Ala Gly Gly Ile Asp Ile Ala
            820                 825                 830

Ser Arg Lys Gly Glu Arg Pro Ala Leu Thr Phe Ile Thr Pro Leu Ala
        835                 840                 845

Ala Pro Gly Glu Glu Gln Arg Arg Arg Thr Lys Thr Gly Lys Ser Glu
    850                 855                 860

Phe Thr Thr Phe Val Glu Ile Val Gly Lys Gln Asp Arg Trp Arg Ile
865                 870                 875                 880

Arg Asp Gly Ala Ala Asp Thr Thr Ile Asp Leu Ala Lys Val Val Ser
                885                 890                 895

Gln Leu Val Asp Ala Asn Gly Val Leu Lys His Ser Ile Lys Leu Asp
            900                 905                 910

Val Ile Gly Gly Asp Gly Asp Asp Val Val Leu Ala Asn Ala Ser Arg
        915                 920                 925

Ile His Tyr Asp Gly Gly Ala Gly Thr Asn Thr Val Ser Tyr Ala Ala
    930                 935                 940

Leu Gly Arg Gln Asp Ser Ile Thr Val Ser Ala Asp Gly Glu Arg Phe
945                 950                 955                 960

Asn Val Arg Lys Gln Leu Asn Asn Ala Asn Val Tyr Arg Glu Gly Val
                965                 970                 975

Ala Thr Gln Thr Thr Ala Tyr Gly Lys Arg Thr Glu Asn Val Gln Tyr
            980                 985                 990

Arg His Val Glu Leu Ala Arg Val  Gly Gln Leu Val Glu  Val Asp Thr
        995                 1000                 1005

Leu Glu  His Val Gln His Ile  Ile Gly Gly Ala Gly  Asn Asp Ser
    1010                 1015                 1020

Ile Thr  Gly Asn Ala His Asp  Asn Phe Leu Ala Gly  Gly Ser Gly
    1025                 1030                 1035

Asp Asp  Arg Leu Asp Gly Gly  Ala Gly Asn Asp Thr  Leu Val Gly
    1040                 1045                 1050

Gly Glu  Gly Gln Asn Thr Val  Ile Gly Gly Ala Gly  Asp Asp Val
    1055                 1060                 1065

Phe Leu  Gln Asp Leu Gly Val  Trp Ser Asn Gln Leu  Asp Gly Gly
    1070                 1075                 1080

Ala Gly  Val Asp Thr Val Lys  Tyr Asn Val His Gln  Pro Ser Glu
    1085                 1090                 1095

Glu Arg  Leu Glu Arg Met Gly  Asp Thr Gly Ile His  Ala Asp Leu
    1100                 1105                 1110

Gln Lys  Gly Thr Val Glu Lys  Trp Pro Ala Leu Asn  Leu Phe Ser
    1115                 1120                 1125

Val Asp  His Val Lys Asn Ile  Glu Asn Leu His Gly  Ser Arg Leu
    1130                 1135                 1140

Asn Asp  Arg Ile Ala Gly Asp  Asp Gln Asp Asn Glu  Leu Trp Gly
    1145                 1150                 1155

His Asp  Gly Asn Asp Thr Ile  Arg Gly Arg Gly Gly  Asp Asp Ile
    1160                 1165                 1170

Leu Arg  Gly Gly Leu Gly Leu  Asp Thr Leu Tyr Gly  Glu Asp Gly
    1175                 1180                 1185

Asn Asp Ile Phe Leu Gln Asp Asp Glu Thr Val Ser Asp Asp Ile
    1190                1195                1200

Asp Gly Gly Ala Gly Leu Asp Thr Val Asp Tyr Ser Ala Met Ile
    1205                1210                1215

His Pro Gly Arg Ile Val Ala Pro His Glu Tyr Gly Phe Gly Ile
    1220                1225                1230

Glu Ala Asp Leu Ser Arg Glu Trp Val Arg Lys Ala Ser Ala Leu
    1235                1240                1245

Gly Val Asp Tyr Tyr Asp Asn Val Arg Asn Val Glu Asn Val Ile
    1250                1255                1260

Gly Thr Ser Met Lys Asp Val Leu Ile Gly Asp Ala Gln Ala Asn
    1265                1270                1275

Thr Leu Met Gly Gln Gly Gly Asp Asp Thr Val Arg Gly Gly Asp
    1280                1285                1290

Gly Asp Asp Leu Leu Phe Gly Gly Asp Gly Asn Asp Met Leu Tyr
    1295                1300                1305

Gly Asp Ala Gly Asn Asp Thr Leu Tyr Gly Gly Leu Gly Asp Asp
    1310                1315                1320

Thr Leu Glu Gly Gly Ala Gly Asn Asp Trp Phe Gly Gln Thr Gln
    1325                1330                1335

Ala Arg Glu His Asp Val Leu Arg Gly Gly Asp Gly Val Asp Thr
    1340                1345                1350

Val Asp Tyr Ser Gln Thr Gly Ala His Ala Gly Ile Ala Ala Gly
    1355                1360                1365

Arg Ile Gly Leu Gly Ile Leu Ala Asp Leu Gly Ala Gly Arg Val
    1370                1375                1380

Asp Lys Leu Gly Glu Ala Gly Ser Ser Ala Tyr Asp Thr Val Ser
    1385                1390                1395

Gly Ile Glu Asn Val Val Gly Thr Glu Leu Ala Asp Arg Ile Thr
    1400                1405                1410

Gly Asp Ala Gln Ala Asn Val Leu Arg Gly Ala Gly Gly Ala Asp
    1415                1420                1425

Val Leu Ala Gly Gly Glu Gly Asp Asp Val Leu Leu Gly Gly Asp
    1430                1435                1440

Gly Asp Asp Gln Leu Ser Gly Asp Ala Gly Arg Asp Arg Leu Tyr
    1445                1450                1455

Gly Glu Ala Gly Asp Asp Trp Phe Phe Gln Asp Ala Ala Asn Ala
    1460                1465                1470

Gly Asn Leu Leu Asp Gly Gly Asp Gly Arg Asp Thr Val Asp Phe
    1475                1480                1485

Ser Gly Pro Gly Arg Gly Leu Asp Ala Gly Ala Lys Gly Val Phe
    1490                1495                1500

Leu Ser Leu Gly Lys Gly Phe Ala Ser Leu Met Asp Glu Pro Glu
    1505                1510                1515

Thr Ser Asn Val Leu Arg Asn Ile Glu Asn Ala Val Gly Ser Ala
    1520                1525                1530

Arg Asp Asp Val Leu Ile Gly Asp Ala Gly Ala Asn Val Leu Asn
    1535                1540                1545

Gly Leu Ala Gly Asn Asp Val Leu Ser Gly Gly Ala Gly Asp Asp
    1550                1555                1560

Val Leu Leu Gly Asp Glu Gly Ser Asp Leu Leu Ser Gly Asp Ala
    1565                1570                1575

Gly Asn Asp Asp Leu Phe Gly Gly Gln Gly Asp Asp Thr Tyr Leu
    1580                1585                1590

Phe Gly Val Gly Tyr Gly His Asp Thr Ile Tyr Glu Ser Gly Gly
    1595                1600                1605

Gly His Asp Thr Ile Arg Ile Asn Ala Gly Ala Asp Gln Leu Trp
    1610                1615                1620

Phe Ala Arg Gln Gly Asn Asp Leu Glu Ile Arg Ile Leu Gly Thr
    1625                1630                1635

Asp Asp Ala Leu Thr Val His Asp Trp Tyr Arg Asp Ala Asp His
    1640                1645                1650

Arg Val Glu Ile Ile His Ala Ala Asn Gln Ala Val Asp Gln Ala

1655                1660                1665

Gly Ile Glu Lys Leu Val Glu Ala Met Ala Gln Tyr Pro Asp Pro
    1670                1675                1680

Gly Ala Ala Ala Ala Ala Pro Pro Ala Ala Arg Val Pro Asp Thr
    1685                1690                1695

Leu Met Gln Ser Leu Ala Val Asn Trp Arg
    1700                1705

<210>  7
<211>  38
<212>  DNA
<213>  artificial

<220>
<223>  primer 1, Example 1

<400>  7
cctgagcacg gtggggccgg ccgtgtcgat cgccgcgg                              38

<210>  8
<211>  52
<212>  DNA
<213>  artificial

<220>
<223>  primer 2, Example 1

<400>  8
gatcgccgcg gcggctagcg tggtaggggc cccgccggcg gtggtcactt cc              52